# EUROPEAN PATENT APPLICATION

(11) **EP 3 998 066 A1**
(43) Date of publication of application: **18.05.2022**
(21) Application number: 21191811.5
(22) Date of filing: 15.04.2016
(51) Int. Cl.: A61K 31/165, A61K 31/60, A61P 9/00, A61P 29/00, A61P 7/02, A61P 9/10, A61P 19/06, A61P 25/28, A61P 27/02, A61P 29/02, A61P 43/00

(54) **COLCHICINE SALICYLATE AND USES THEREOF**

(30) Priority: 17.04.2015 US 201562148985 P
(62) Divisional of application: 16718734.3
(71) Applicant: Murray And Poole Enterprises Limited, Gibraltar GX111AA (GB)
(72) Inventor: RIEL, Susanne, Dubai (AE)
(74) Representative: FRKelly

(57) **Abstract**

Pharmaceutical compositions of colchicine salicylate, including once-a-day orally administered formulations are provided. Method of treating and/or preventing cardiovascular disease and/or inflammatory disease in mammalian subjects comprising the administration of the novel formulations disclosed herein is also provided.

## Description

### RELATED APPLICATIONS

This application claims the benefit of U.S. Provisional Application No. 62/148,985, filed April 17, 2015, the entire contents of which are incorporated herein by reference.

### BACKGROUND OF THE INVENTION

Colchicine, chemical name (―)-N-[(7S, 12aS)-1,2,3,10-tetramethoxy-9-oxo-5,6,7,9-tetrahydrobenzo[a]heptalen-7-yl]-acetamide, is an alkaloid found in extracts of Colchicum autumnale, Gloriosa superba, and other plants. It is a microtubule-disrupting agent used in the treatment of conditions that may be treated, relieved or prevented with anti-inflammatory treatment.

Colchicine is well recognized as a valid therapy in acute flares of gouty arthritis, familial Mediterranean fever (FMF), and Behçet's disease. It has also been used to treat many inflammatory disorders prone to fibrosis. In the recent past, colchicine has been proposed to be effective in therapy in cardiovascular diseases.

In particular, colchicine has been proposed as a first treatment option for recurrent pericarditis (class I indication) and optional for acute pericarditis (class IIa indication) in the 2004 European guidelines on the management of pericardial diseases (Maisch et al., Guidelines on the Diagnosis and Management of Pericardial Diseases, Eur Heart J., 2004, 25, 916-928).

Imazio et al. (Circulation, 2005, 112 (13), 2012-2016) showed that colchicine was effective for the treatment and the prevention of recurrent pericarditis in a prospective, randomized, open-label designed study of 120 patients with a first episode of acute pericarditis (idiopathic, viral, postpericardiotomy syndromes, and connective tissue diseases), who were randomly assigned to conventional treatment with aspirin or conventional treatment plus colchicine (1.0 to 2.0 mg for the first day and then 0.5 to 1.0 mg/day for 3 months). The primary end point was recurrence rate, which was significantly reduced from 32.3% down to 10.7% at 18 months in the colchicine group (p=0.004).

Further, the same group showed that colchicine could be efficient after conventional treatment failure to manage acute pericarditis (Imazio at al., Arch InternMed, 2005, 165 (17), 1987-91). In a prospective, randomized, open-label design, 84 consecutive patients with a first episode of recurrent pericarditis were randomly assigned to receive conventional treatment with aspirin alone or conventional treatment plus colchicine (1.0-2.0 mg the first day and then 0.5-1.0 mg/d for 6 months). The primary end point was the recurrence rate, which was significantly decreased in the colchicine group (actuarial rates at 18 months were 24.0% vs 50.6% with conventional treatment).

It has also been shown that colchicine is effective for secondary prevention of recurrent pericarditis Imazio et al., Ann. Intern. Med., 2011, 155 (7), 409-14). Colchicine has also been proposed to reduce postpericardiotomy reactions revealed as pericarditis (Imazio et al., Am. Heart J., 2011, 162 (3), 527-532; Meurin and Tabet, Arch. Cardiovasc. Dis., 2011, 104 (8-9), 425-427).

Colchicine for the treatment of post-pericardiotomy syndrome (PPS) was tested for the first time in a preliminary prospective, open-label, randomized trial of colchicine (1.5 mg/day) compared with placebo beginning on the third post-operative day in 163 patients who underwent cardiac surgery (Finkelstein et al., Herz, 2002 27, 791-194).

The effectiveness of colchicine for the prevention of PPS has also been shown in a multicentre, double-blind, randomized trial, in which 360 patients (mean age 65.7+12.3 years, 66% males), 180 in each treatment arm, were randomized to receive placebo or colchicine (1.0 mg twice daily for the first day followed by a maintenance dose of 0.5 mg twice daily for 1 month in patients ≥70 kg, and halved doses for patients, 70 kg or intolerant to the highest dose) on the third post-operative day (Imazio et al., European Heart Journal, 2010, 31, 2749-2754).

In another study, the effectiveness of colchicine has been shown for cardiovascular disease. In this clinical trial with a prospective, randomized, observer-blinded endpoint design, 532 patients with stable coronary disease receiving aspirin and/or clopidogrel (93%) and statins (95%) were randomly assigned colchicine 0.5 mg/day or no colchicine and followed for a median of 3 years (Nidorf et al., JACC, 2013, 61 (4), 404-410). This study showed that colchicine 0.5 mg/day administered in addition to statins and other standard secondary prevention therapies appeared effective for the prevention of cardiovascular events in patients with stable coronary disease.

For the treatment of gout, the recommended dose of colchicine (COLCRYS^{®}) is 1.8 mg/day in one or multiple doses in one hour. For adults with gout, treatment is initiated with a dose of 1.2 mg at the first sign of symptoms followed by 0.6 mg one hour later. (Physician's Desk Reference, 68th ed., (2014)).

COLCRYS^{®} is an immediate release formulation. Adverse effects associated with the administration of COLCRYS^{®} include, but are not limited to, nausea, vomiting, abdominal pain, diarrhea, hair loss, weakness, nerve irritation, severe anemia, low white blood counts, and low platelets (Physician's Desk Reference, 68th ed., (2014)).

The instant invention addresses these and other needs by providing a modified derivative of colchicine. This invention additionally provides an effective, once-daily dosage form of colchicine salicylate, which may improve patient compliance, increase bioavailability, and reduce some of the side effects of colchicine compared to the current or higher daily doses of immediate release colchicine formulations.

### BRIEF SUMMARY OF THE INVENTION

According to aspects of the invention described herein, there is provided formulations of colchicine salicylate for use in treating and/or preventing cardiovascular and/or inflammatory diseases/conditions.

### DETAILED DESCRIPTION OF THE INVENTION

### I. Definitions

For the purposes of this invention, the term "colchicine" includes colchicine and colchicine salicylate, and further refers to pharmaceutically acceptable solvates, including hydrates, and co-crystals of such compounds and such salts. All forms of such derivatives of colchicine are contemplated herein, including all crystalline, amorphous, and polymorph forms.

"Pharmacokinetic parameters" describe the in vivo characteristics of an active agent (or a metabolite or a surrogate marker for the active agent) over time, such as plasma concentration (C), Cmax, Cn, C24, Tmax, and AUC. "Cmax" is the measured plasma concentration of the active agent at the point of maximum, or peak, concentration. "Cmin" is the measured plasma concentration of the active agent at the point of minimum concentration. "Cn" is the measured plasma concentration of the active agent at about n hours after administration. "C24" is the measured plasma concentration of the active agent at about 24 hours after administration. The term "Tmax" refers to the time at which the measured plasma concentration of the active agent is the highest after administration of the active agent. "AUC" is the area under the curve of a graph of the measured plasma concentration of an active agent vs. time, measured from one time point to another time point. For example AUC0-t is the area under the curve of plasma concentration versus time from time 0 to time t, where t can be the last time point with measurable plasma concentration for an individual formulation. The AUC0-∞ or AUC0-INF is the calculated area under the curve of plasma concentration versus time from time 0 to time infinity. In steady-state studies, AUC0-τ is the area under the curve of plasma concentration over the dosing interval (i.e., from time 0 to time τ (tau), where tau is the length of the dosing interval. Other pharmacokinetic parameters are the parameter Ke or Kel, the terminal elimination rate constant calculated from a semi-log plot of the plasma concentration versus time curve; tl/2 the terminal elimination half-life, calculated as 0.693/Kel; CL/F denotes the apparent total body clearance after administration, calculated as Total Dose/Total AUC∞; and Varea/F denotes the apparent total volume of distribution after administration, calculated as Total Dose/(Total AUC∞×Kel).

"Efficacy" means the ability of an active agent administered to a patient to produce a therapeutic effect in the patient.

"Bioavailability" means the extent or rate at which an active agent is absorbed into a living system or is made available at the site of physiological activity. For active agents that are intended to be absorbed into the bloodstream, bioavailability data for a given formulation may provide an estimate of the relative fraction of the administered dose that is absorbed into the systemic circulation. "Bioavailability" can be characterized by one or more pharmacokinetic parameters. An increase in bioavailability refers to a beneficial change in one or more pharmacokinetic parameters that characterize the bioavailability of colchicine. The increase in bioavailability can be from about 1 % to about 50%. In this range, useful values include from about 2% to about 40%. The increase can be 2%, 3%, 4%, 5%, 6%, 7%, 8%, 9%, 10%, 11%, 12%, 13%, 14%, 16%, 17%, 18%, 19%, 20%, 21%, 22%, 23%, 24%, 25%, 26%, 27%, 28%, 29%, 30% or more.

A "dosage form" means a unit of administration of an active agent. Examples of dosage forms include tablets, capsules, injections, suspensions, liquids, emulsions, creams, ointments, suppositories, inhalable forms, transdermal forms, and the like.

An "immediate release formulation" refers to a formulation that releases greater than or equal to about 80% of the pharmaceutical agent in less than or equal to about 30 min.

For the purposes of this application, an enhancing agent ("enhancer") is defined as any non-pharmaceutically active ingredient that improves the therapeutic potential of a formulation.

"Sustained release," also referred to herein as "extended release," is defined herein as release of a pharmaceutical agent in a continuous manner over a prolonged period of time.

By "prolonged period of time" it is meant a continuous period of time of greater than about 1 hour, greater than about 4 hours, greater than about 8 hours, greater than about 12 hours, greater than about 16 hours, or up to more than about 24 hours.

The term "delayed release" is used in its conventional sense to refer to a drug formulation in which there is a time delay provided between administration of a drug dosage form and the release of the drug therefrom. After the initial delay, the delayed release may or may not involve gradual release of drug over an extended period of time, and thus may or may not also include a "sustained release." Non-limiting examples of "delayed release" formulations are enterically coated compositions.

The term "controlled release" refers to a drug-containing formulation or fraction thereof in which release of the drug is not immediate after administration, i.e., with a "controlled release" or "sustained release" formulation, administration does not result in immediate release of greater than or equal to about 80% of the pharmaceutical agent in less than or equal to about 30 min. However, in an enteric composition, for example, once the enteric coating erodes in the intestines, the release profile may be an immediate release or a sustained release of all the active agent in the intestines.

As used herein, unless otherwise noted, "rate of release" or "release rate" or "dissolution rate" of a drug refers to the quantity of drug released from a dosage form per unit time, e.g., milligrams of drug released per hour (mg/hr) or a percentage of a total drug dose released per hour. Drug release rates for dosage forms are typically measured as an in vitro rate of drug release, i.e., a quantity of drug released from the dosage form per unit time measured under appropriate conditions and in a suitable fluid. The release rates referred to herein are determined by placing a dosage form to be tested in a medium in an appropriate dissolution bath. Aliquots of the medium, collected at pre-set intervals, are then injected into a chromatographic system fitted with an appropriate detector to quantify the amounts of drug released during the testing intervals.

Side effect is defined herein as a secondary and usually adverse effect of a drug.

Terms such as "treating" or "treatment" or "to treat" or "alleviating" or "to alleviate" refer to both 1) therapeutic measures that cure, slow down, lessen symptoms of, reverse, and/or halt progression of a diagnosed pathologic condition or disorder and 2) prophylactic or preventative measures that prevent and/or slow the development of a targeted pathologic condition or disorder. Thus those in need of treatment include those already with the disorder; those prone to have the disorder; and those in whom the disorder is to be prevented. Beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

By "subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sports, or pet animals such as dogs, cats, guinea pigs, rabbits, rats, mice, horses, cattle, cows, bears, and so on. The meaning of the terms "eukaryote", "animal", "mammal", etc. is well known in the art and can, for example, be deduced from Wehner und Gehring (1995; Thieme Verlag). In the context of this invention, it is also envisaged that animals are to be treated which are economically, agronomically or scientifically important. Scientifically important organisms include, but are not limited to, mice, rats, and rabbits. Non-limiting examples of agronomically important animals are sheep, cattle and pigs, while, for example, cats and dogs may be considered as economically important animals. In one embodiment, the subject/patient is a mammal; in another embodiment, the subject/patient is a human or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orang-utan, a gibbon, a sheep, cattle, or a pig); most preferably, the subject/patient is a human.

As used herein "a subject in need thereof" includes subjects having one or more of the cardiovascular and/or inflammatory diseases or conditions described herein. A subject in need thereof includes a subject that has been diagnosed as suffering from a cardiovascular and/or inflammatory condition intended to be treated. A subject in need thereof includes a subject that has not been diagnosed as suffering from a cardiovascular and/or inflammatory condition intended to be treated but is susceptible or predisposed to such a disease or condition. Accordingly, as described herein, colchicine salicylate can be administered therapeutically or prophylactically to a subject in need thereof.

### II. Colchicine and Colchicine Derivatives

In the following, colchicine used according to the present invention will be described in detail. The chemical structure of colchicine ***(ChemID 2012)*** is as follows:

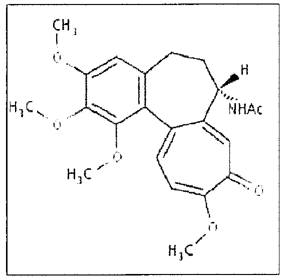

The chemical name of colchicine is: N[5,6,7,9-tetrahydro-1,2,3,10-tetratmethoxy 9-oxobenzo[a]heptalen-7-yl],(S)-acetamide; molecular formula:C₂₂H₂₅NO₆; CAS number: 64-86-8.

Colchicine is an anti-inflammatory drug with a long history in human medicine, used for the symptomatic treatment of inflammatory diseases, most prominently gout. It is a natural product which can be extracted from two plants of the lily family, Colchicum autumnale and Gloriosa superba. Colchicine is a tricyclic alkaloid and has a molecular mass of 399.437. The active ingredient colchicine as well as its tablet formulation is listed in various national and international pharmacopeias such as the United States Pharmacopeia (USP).

The positive effect of its plant source in the treatment of rheumatism and swelling was described first around 1500 B.C. in Egypt. Its use in gout was first described around 1500 years ago (Graham and Roberts, 1953, Ann Rheum Dis 12(1): 16-9). Today, the therapeutic value of colchicine is well established in a number of inflammatory diseases and approved by FDA for the prophylaxis and treatment of acute gout flares and familial Mediterranean fever (FMF). Other important established, though off-label uses are amongst others, Behcet's disease and recurrent pericarditis. In all known indications, it is generally administered orally as solid tablets in strengths of 0.5-0.6mg/tablet (e.g. Europe and United States, respectively).

The pharmacotherapeutic mechanism of action of colchicine in diverse disorders is not fully understood, though it is known that the drug accumulates preferentially in leucocytes, particularly neutrophils which is important for its therapeutic effect. Three major interactions of colchicine with specific proteins modulate its pharmacokinetics: tubulin, cytochrome P450 3A4 (CYP3A4), and P-glycoprotein. It is assumed that most therapeutic effects of the drug are related to its capacity to bind to β-tubulin, thus inhibiting self-assembly and polymerization of microtubules. Availability of tubulin is essential for several cellular functions such as mitosis. Therefore colchicine effectively functions as a "mitotic poison" or spindle poison. By inhibiting microtubule self-assembly, colchicine interferes with many cellular functions involved in the immune response such as modulation of the production of chemokines and prostanoids and inhibition of neutrophil and endothelial cell adhesion molecules. Eventually it decreases neutrophil degranulation, chemotaxis and phagocytosis, thus reducing the initiation and amplification of inflammation. Colchicine also inhibits uric acid crystal deposition (a process important to the genesis of gout), which is enhanced by a low pH in the tissues, probably by inhibiting oxidation of glucose and subsequent lactic acid reduction in leukocytes *(*Imazio, Brucato et al. 2009, Eur Heart J,30(5): 532-9; Cocco, Chu et al. 2010, Eur J Intern Med, 21(6): 503-8; Stanton, Gernert el al. 2011, Med Res Rev, 31 (3): 443-81*)*. In the management of pericarditis, colchicine excerpts its therapeutic effect by suppressing the acute pericardial inflammation. However, the exact cellular and molecular mechanisms of how colchicine relieves pain and inflammation in acute pericarditis and prevents recurrences are not fully understood.

Colchicine may also be modified to yield a derivative such as colchicine salicylate. Colchicine salicylate may be combined with another pharmaceutically acceptable salt form of colchicine, wherein the colchicine is modified by making acid or base addition salts thereof, and further refers to pharmaceutically acceptable solvates, including hydrates, polymorphs, and co-crystals of such compounds and such salts. Examples of pharmaceutically acceptable salts include, but are not limited to, mineral or organic acid addition salts of basic residues such as amines; alkali or organic addition salts of acidic residues; and the like, and combinations comprising one or more of the foregoing salts. The pharmaceutically acceptable salts include non-toxic salts and the quaternary ammonium salts of the colchicine. For example, non-toxic acid salts include those derived from inorganic acids such as hydrochloric, hydrobromic, sulfuric, sulfamic, phosphoric, nitric and the like; other acceptable inorganic salts include metal salts such as sodium salt, potassium salt, cesium salt, and the like; and alkaline earth metal salts, such as calcium salt, magnesium salt, and the like, and combinations comprising one or more of the foregoing salts. Pharmaceutically acceptable organic salts includes salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC—(CH2)n—COOH where n is 0-4, and the like; organic amine salts such as triethylamine salt, pyridine salt, picoline salt, ethanolamine salt, triethanolamine salt, dicyclohexylamine salt, N,N'-dibenzylethylenediamine salt, and the like; and amino acid salts such as arginate, asparaginate, glutamate, and the like; and combinations comprising one or more of the foregoing salts; organic amine salts such as triethylamine salt, pyridine salt, picoline salt, ethanolamine salt, triethanolamine salt, dicyclohexylamine salt, N,N' dibenzylethylenediamine salt, and the like; and amino acid salts such as arginate, asparaginate, glutamate, and the like; and combinations comprising one or more of the foregoing salts. All forms of such derivatives of colchicine are contemplated herein, including all crystalline, amorphous, and polymorph forms. Specific colchicine salts include colchicine salicylate, colchicine hydrochloride, colchicine dihydrochloride, and co-crystals, hydrates or solvates thereof.

In one embodiment, the colchicine derivative is colchicine salicylate, which is a salt of a base found in colchicum, corm and seeds.

The chemical structure of colchicine salicylate *(**ChemID 2015**)* is as follows:

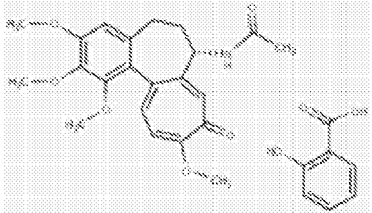

The molecular formula for colchicine salicylate is C₂₂H₂₅NO₆,C₇H₆O₃ or C₂₉H₃₁NO₉. Colchicine salicylate in the context of the present invention can be used for the prevention and/or treatment of cardiovascular diseases and/or inflammatory diseases.

### III. Colchicine Salicylate Formulations

The present invention additionally provides a pharmaceutical composition comprising colchicine salicylate, for the treatment or prevention of a cardiovascular disease and/or an inflammatory disorder in a subject. The colchicine salicylate formulation of the present invention may be formulated in a dosage form selected from a tablet, a pill, a capsule, a caplet, a troche, a sachet, a cachet, a pouch, sprinkles, or any other form suitable for oral administration. Such preparations can in principal be in any form conceivable to the skilled person and include pharmaceutical forms for oral (solid, semi-solid, liquid), dermal (dermal patch), sublingual, parenteral (injection), ophthalmic (eye drops, gel or ointment) or rectal (suppository) administration.

According to the present invention, the colchicine salicylate composition as described herein (i.e., inter alia, in the form of a (pharmaceutical) composition) can be administered in the form of an immediate release preparation where the formulation releases greater than or equal to about 80% of the pharmaceutical agent in less than or equal to about 1 hour. Alternatively, the colchicine salicylate composition of the present invention can be administered in the form of a sustained release preparation. In a sustained release preparation, the colchicine salicylate is released from the formulation at a sustained rate along a pre-determined or desired release profile. Such release is achieved by incorporation into the formulation of an extended release component and an optional immediate release component. The colchicine salicylate composition may be in the form of a delayed release preparation. The term "delayed release" is used in its conventional sense to refer to a drug formulation in which there is a time delay provided between administration of a drug dosage form and the release of the drug therefrom.

In accordance with the present invention, immediate release preparations encompass all pharmaceutical forms that release the drug substance over a short period of time following application to the patient and are not designed to delay or prolong the release of the drug substance. Such a short period of time may be between 5, 10, 15, 20, 25, 30, 35, 40, 45, 50, 55, 60, 65, 70, 75, 80, 85 and 90 minutes. Immediate release as used herein may also be defined as making colchicine available to the patient regardless of uptake, as some colchicine may never be absorbed by the patient. Various immediate release dosage forms may be designed readily by one of skill in art as disclosed herein to achieve delivery and sustained release of colchicine to the liver and/or both the small and large intestines, to only the small intestine, or to only the large intestine.

In accordance with the present invention, sustained release preparations encompass all pharmaceutical forms that create a steady drug release profile making the drug substance available over an extended period of time following application to the patient. Such an extended period of time may be between 10, 20, 30, 40, 50 or 60 minutes and about 2, 3, 4, 5, 6, 7, 8, 9, 10, 11, 12, 13, 14, 15, 16, 17, 18, 19, 20, 21, 22, 23 or 24 hours. Extended release may also be defined functionally as the release of over 50, 55, 60, 65, 70, 75, 80, 85, 90, 95 or 99 percent (%) of colchicine after about 10, 20, 30, 40, 50 or 60 minutes and about 2, 3, 4, 5, 6, 7, 8, 9, 10, 11, 12, 13, 14, 15, 16, 17, 18, 19, 20, 21, 22, 23 or 24 hours. Extended release as used herein may also be defined as making colchicine or its derivative available to the patient regardless of uptake, as some colchicine or its derivative may never be absorbed by the patient. Various extended release dosage forms may be designed readily by one of skill in art as disclosed herein to achieve delivery and sustained release of colchicine to the liver and/or both the small and large intestines, to only the small intestine, or to only the large intestine.

In some embodiments, the colchicine salicylate preparations may be pH independent. This allows such preparations to dissolve in almost any environment. In other embodiments, the colchicine salicylate preparations may be pH dependent. This allows release to be accomplished at some generally predictable location in the lower intestinal tract more distal to that which would have been accomplished if there had been no delayed release alterations. A method for delay of release is, e.g., a coating. Any coatings should be applied to a sufficient thickness such that the entire coating does not dissolve in the gastrointestinal fluids at pH below about 5, but does dissolve at pH about 5 and above. It is expected that any anionic polymer exhibiting a pH-dependent solubility profile can be used as an enteric coating in the practice of the present invention to achieve delivery to the lower gastrointestinal tract. Polymers and compatible mixtures thereof may be used to provide the coating for the delayed or the extended release of active ingredients, and some of their properties, include, but are not limited to: shellac, also called purified lac, a refined product obtained from the resinous secretion of an insect. This coating dissolves in media of pH >7.

In some embodiments, the colchicine salicylate preparations may be influenced by the presence of alcohol in the body. The presence of alcohol in a patient's body can increase dissolution of the composition and can cause immediate release of the entire dose. This effect is known as "dose dumping" and is dependent on the alcohol solubility of the materials. For sustained release preparations which contain a higher dose for slow release over 24 hours, for instance, this effect can have safety concerns and can even be life threatening.

To achieve a uniform or continuous rate of release, sustained release preparations may be prepared using time release hydrophilic matrices. These time release hydrophilic matrices are known in the field of drug formulations. For example, one such hydrophilic matrix is hydroxypropyl methylcellulose (HPMC) or Hypromellose. Hydrophilic matrices provide an initial release of the drug product in the initial phase mainly triggered by a rapid swelling of the surface of the matrix tablet, combined with an erosion process leading to an immediate release of the drug substance distributed close to the surface of the tablet. In an embodiment, about 50%, about 45%, about 40%, about 35%, about 30%, about 25%, about 20%, about 15%, or about 10% of the drug substance may immediately be released depending on the desired release profile. In an embodiment, at least about 20% of the drug substance may immediately be released. In another embodiment, at least about 20% of the drug substance may be released within about the first 30 minutes.

As used herein, the term "about" or "approximately" refers to a variation of 10% from the indicated values (e.g., 50%, 45%, 40%, etc.), or in case of a range of values, means a 10% variation from both the lower and upper limits of such ranges. For instance, "about 50%" refers to a range of between 45% and 55%.

Within the initial swelling of the tablet surface a gel formation of the hydrophilic matrix starts. This gelling prevents the tablet core from dissolving and disintegrating immediately, thereby allowing the main part of the drug substances to dissolve slowly over time within in this gel structure and diffuse into solution following the rules of Fick's law. The diffusion itself may be triggered in this formulation approach by the concentration of the Hypromellose and the viscosity of the formed gel, defined over the molecular weight of the Hypromellose. Therefore, drug release profiles can be modified by varying different viscosity grades of Hypromellose or mixtures thereof. All corresponding formulation and process parameters achieving the predicted release profile are common knowledge and can be adjusted using actual development technologies e.g. formulation screenings, statistical trials designs.

In an embodiment, the substance responsible for sustained release of the controlled-release formulation can further mix with a binder. The binder is added to increase the mechanical strength of the granules and tablets during formation. Binders can be added to the formulation in different ways: (1) as a dry powder, which is mixed with other ingredients before wet agglomeration, (2) as a solution, which is used as agglomeration liquid during wet agglomeration, and is referred to as a solution binder, and (3) as a dry powder, which is mixed with the other ingredients before compaction. In this form the binder is referred to as a dry binder. Solution binders are a common way of incorporating a binder into granules. In certain embodiments, the binder used in the formulation is in the form of a dry powder binder. Non-limiting examples of binders useful for the core include hydrogenated vegetable oil, castor oil, paraffin, higher aliphatic alcohols, higher aliphatic acids, long chain fatty acids, fatty acid esters, wax-like materials such as fatty alcohols, fatty acid esters, fatty acid glycerides, hydrogenated fats, hydrocarbons, normal waxes, stearic acid, stearyl alcohol, hydrophobic and hydrophilic polymers having hydrocarbon backbones, and mixtures thereof. Specific examples of water-soluble polymer binders include modified starch, gelatin, polyvinylpyrrolidone, cellulose derivatives (such as for example hydroxypropyl methylcellulose (HPMC) and hydroxypropyl cellulose (HPC)), polyvinyl alcohol and mixtures thereof. In an embodiment, the binder is HPMC. In another embodiment, the binder is Hypromellose 6mPa^{∗}s. In an embodiment, the binder can be present in an amount of from about 1% to about 30% by weight of the formulation.

In another embodiment of the invention, the colchicine salicylate formulation may include a disintegrant. A disintegrant refers to an agent used in pharmaceutical preparation of tablets, which causes them to disintegrate and release their medicinal substances on contact with moisture. In an embodiment, the disintegrant may be water soluble to support the disintegrantation of a tablet in the stomach. Non-limiting examples of disintegrants for use in the formulation include sucrose, lactose, in particular lactose monohydrate, trehalose, maltose, mannitol and sorbitol, croscarmellose sodium, crospovidone, alginic acid, sodium alginate, methacrylic acid DVB, microcrystalline cellulose, polacrilin potassium, sodium starch glycolate, starch, pregelatinized starch and mixtures thereof. In at least one embodiment the disintegrant is selected from microcrystalline cellulose (e.g. Avicel PH101), cross-linked polyvinylpyrrolidone (e.g. KOLLIDON^{®} CL), cross-linked sodium carboxymethylcellulose (e.g. AC-DI-SOL(TM)), microfine cellulose, starch or starch derivatives such as sodium starch glycolate (e.g. EXPLOTAB^{®}), pregelatinised starch, maize starch, potato starch, rice starch, wheat starch, or combinations with starch (e.g. PRIMOJEL(TM)), swellable ion-exchange resins, such as AMBERLITE(TM) IRP 88, formaldehyde-casein (e.g. ESMA SPRENG(TM)), and others (such as cross linked polyvinylpyrrolidone, cationic exchange resin), and mixtures thereof.

In another embodiment of the invention, the colchicine salicylate formulation may include a filling agent or filler. A filling agent refers to an inert substance used as filler to create desired bulk, flow properties, and compression characteristics in preparation of tablets. Non-limiting examples of filling agents for use in the formulation include sucrose, lactose, in particular lactose monohydrate, trehalose, maltose, mannitol and sorbitol, croscarmellose sodium, crospovidone, alginic acid, sodium alginate, methacrylic acid DVB, cross-linked PVP, microcrystalline cellulose, polacrilin potassium, sodium starch glycolate, starch, pregelatinized starch and mixtures thereof. In an embodiment, lactose monohydrate is included as a filling agent in an amount of about 10% to about 80%, preferably about 59%, by weight of the tablet. In an embodiment, pregelatinized starch is included as a filling agent in an amount of about 5% to about 50%, preferably about 7.5%, by weight of the tablet.

In another embodiment, the sustained release formulation of the present invention may include a release retarding agent for maintaining a uniform release rate of the drug. Examples of retarding agents include, but are not limited to, cellulose ethers, cellulose esters, acrylic acid copolymers, waxes, gums, glyceryl fatty acid esters and sucrose fatty acid esters. In one embodiment, the retarding agent is RETALAC^{®} (Meggle), a spray agglomerated blend of 50 parts lactose monohydrate and 50 parts hypromellose. The viscosity of hypromellose used herein may range from 6 mPa^{∗}s ― 100,000 mPa^{∗}s. In an embodiment, the viscosity of hypromellose used is 4000 mPa^{∗}s. Adjusting the amount of retarding agent in the composition may alter the release rate of the drug. In one embodiment, the retarding agent of the formulation of the present invention releases colchicine in a continuous and uniform manner and is adjusted in such a way that about 80% of the active ingredient is released in vitro in the predetermined period of time. By way of example, and by no means limiting the scope of the invention, the period of time may be not more than 24 hours, not more than 16 hours, not more than 12 hours, not more than 8 hours, not more than 6 hours, not more than 4 hours, not more than 3.5 hours, or not more than 1.5 hours depending on desired attributes of the final product. It is understood that the release rate can vary based on whether the experiment is conducted in vitro or in vivo. Therefore, if the desired release rate is between about 1.5 to about 3.5 hours in vitro or between about 1.5 to about 6 hours in vitro, the release rate under in vivo conditions, depending on the experimental conditions, may actually be different. In an embodiment, the sustained release formulation of the present invention releases colchicine in a continuous and uniform manner in such a way that about 80% of the active ingredient is released in vitro in between about 1.5 and about 3.5 hours.

In another embodiment, the colchicine salicylate formulation of the present invention may include a glidant. A glidant can be used to improve powder flow properties prior to and during tableting and to reduce caking. Suitable glidants include colloidal silicon dioxide, magnesium trisilicate, powdered cellulose, talc, tribasic calcium phosphate and the like. In one embodiment, talc is included as a glidant in an amount of about 0.05% to about 5%, preferably about 1%, by weight of the tablet.

In another embodiment, the colchicine salicylate formulation of the present invention may include a lubricant. Lubricants can be added to pharmaceutical formulations to decrease any friction that occurs between the solid and the die wall during tablet manufacturing. High friction during tableting can cause a scries of problems, including inadequate tablet quality (capping or even fragmentation of tablets during ejection, and vertical scratches on tablet edges) and may even stop production. Accordingly, lubricants are added to certain tablet formulations of the present invention including certain embodiments of the formulation described herein. Non-limiting examples of lubricants useful for the core include glyceryl behenate, stearic acid, hydrogenated vegetable oils (such as hydrogenated cottonseed oil (STEROTEX^{®}), hydrogenated soybean oil (STEROTEX^{®} HM) and hydrogenated soybean oil & castor wax (STEROTEX^{®} K)), stearyl alcohol, leucine, polyethylene glycol (MW 1450, suitably 4000, and higher), magnesium stearate, glyceryl monostearate, polyethylene glycol, ethylene oxide polymers (for example, available under the registered trademark CARBOWAX^{®} from Union Carbide, Inc., Danbury, Conn.), sodium lauryl sulfate, magnesium lauryl sulfate, sodium oleate, sodium stearyl fumarate, DL-leucine, colloidal silica, mixtures thereof and others as known in the art. In one embodiment, stearic acid is included as a lubricant in an amount of about 0.05% to about 5%, preferably about 1%, by weight of the tablet.

Sweeteners that can also be used in the taste-masking coating of certain embodiments of the matrix dosage forms include glucose (corn syrup), dextrose, invert sugar, fructose, and mixtures thereof (when not used as a carrier); saccharin and its various salts, such as sodium salt; dipeptide sweeteners such as aspartame; dihydrochalcone compounds, glycyrrhizin; Steva Rebaudiana (Stevioside); chloro derivatives or sucrose such as sucralose; and sugar alcohols such as sorbitol, mannitol, xylitol, and the like. Also contemplated are hydrogenated starch hydrolysates and the synthetic sweeteners such as 3,6-dihydro-6-methyl-1-1-1,2,3-oxathiazin-4-1-2,2-dioxide, particularly the potassium salt (acesulfame-K), and sodium and calcium salts thereof. The sweeteners can be used alone or in any combination thereof.

The colchicine salicylate formulation of the present invention can further contain one or more pharmaceutically acceptable excipients such as granulating aids or agents (such as gelatin), colorants, flavorants, pH adjusters, anti-adherents, glidants and like excipients conventionally used in pharmaceutical compositions.

Useful granulation agents include gelatin, copovidone (povidone), starch, starch paste, cellulose derivatives (e.g., HPMC, hydroxypropyl cellulose), acacia, sodium alginate, guar, siliceous material, water-soluble polymeric materials (e.g., polyethylene glycols, such as PEG400 or lower MW), polyvinylpyrrolidone, ethanol or isopropanol, Pluronic L44, and the like.

In an embodiment, a coloring excipient can be advantageously added as giving rise to visual change preventing abuse. It can color simultaneously the liquid or the particles or one independently of the other. Among suitable coloring excipients the following may be cited: indigotine, cochineal carminic acid, yellow orange S, allura red AC, iron oxides, cucurmin, riboflavin, tartrazine, quinoline yellow, azorubine, amaranth, carmines, erythosine, red 2G, patented blue V, glittering blue FCF, chlorophylls, copper complexes of chlorophylls, green S, caramel, glittering black BN, carbo medicinalis vegetabilis, brown FK and HT, carotenoids, Annatto extracts, paprika extracts, lycopene, lutein, canthaxanthin, beetroot red, anthocyanes, calcium carbonate, titanium dioxide, aluminium, silver, gold or litholrubin BK or any other coloring excipient suitable for an oral administration.

In an embodiment, the colchicine salicylate formulation may be coated. Coatings may provide a variety of functions. In some embodiments, coatings may be used, for example, to achieve delayed release, resistance to acid, targeted release in the lower GI tract, avoidance of bad taste in mouth. In some embodiments, coatings may be used to protect the API/tablet from light and provide for better mechanical resistance. Of course it should be appreciated that a coating may serve other functions as well and a person skilled in the art knows the purpose of tablet coating.

The pharmaceutical composition and/or the solid carrier particles can be coated with one or more enteric coatings, seal coatings, film coatings, barrier coatings, compress coatings, fast disintegrating coatings, or enzyme degradable coatings. Multiple coatings may be applied for desired performance. Further, one or more of the actives may be provided for immediate release, pulsatile release, controlled release, extended release, delayed release, targeted release, synchronized release, or targeted delayed release. In fact, the formulation may include combinations of typical pharmaceutical actives (e.g., pseudephedrin) and vitamins (e.g., Vitamin C), minerals (Ca, Mg, Zn, K) or other supplements (e.g., St. John's Wort, echinacae, amino acids). For release/absorption control, solid carriers can be made of various component types and levels or thicknesses of coats, with or without an active ingredient. Such diverse solid carriers can be blended in a dosage form to achieve a desired performance. The liquid formulations may be delivered to, and adapted for, oral, nasal, buccal, ocular, urethral, transmucosal, vaginal, topical or rectal delivery, although oral delivery is used mostly.

When formulated with microparticles or nanoparticles, the drug release profile can easily be adapted by adding a coating, e.g., a hard or soft gelatin coating, a starch coating, a resin or polymer coating and/or a cellulosic coating. Although not limited to microparticles or nanoparticles (as in, e.g., microcapsules or nanocapsules), such dosage forms may be further coated with, for example, a seal coating, an enteric coating, an extended release coating, or a targeted delayed release coating. The term "enteric coating" as used herein relates to a mixture of pharmaceutically acceptable excipients that is applied to, combined with, mixed with or otherwise added to the carrier or composition. The coating may be applied to an active that is compressed, molded or extruded and may also include: gelatin, and/or pellets, beads, granules or particles of the carrier or composition. The coating may be applied through an aqueous dispersion or after dissolving in appropriate solvent. The carrier may or may not be fully or partially biodegradable.

In an embodiment, polymethacrylate acrylic polymers can be employed as coating polymers. In at least one embodiment, the coating is an acrylic resin lacquer used in the form of an aqueous dispersion, such as that which is commercially available from Rohm Pharma under the trade name EUDRAGIT^{®} or from BASF under the trade name KOLLICOAT^{®}. In a more preferable embodiments, EUDRAGIT^{®} E100 is used as the coating polymer, which is a cationic copolymer based on dimethylaminoethyl methacrylate and neutral methacrylic esters having a average molecular weight is approximately 150,000. Different coating polymers of the certain embodiments can be mixed together in any desired ratio in order to ultimately obtain a coating having a desirable drug dissolution profile. Coating methods can consist in spraying a solution of the polymer on the tablets, either in a pan coater or a fluid bed coating apparatus. The solvent may be organic or aqueous, depending on the nature of the polymer used. In a preferable embodiment, the solvent is alcohol. Coating methods are well known in the art.

The compositions of the present invention can also be formulated as enteric coated delayed release oral dosage forms, i.e., as an oral dosage form of a pharmaceutical composition as described herein that uses an enteric coating to effect release in the lower gastrointestinal tract. The enteric coated dosage form will generally include microparticles, microgranules, micropellets or microbeads of the active ingredient and/or other composition components, which are themselves coated or uncoated. The enteric coated oral dosage form may also be a capsule (coated or uncoated) containing pellets, beads or granules of the solid carrier or the composition, which are themselves coated or uncoated.

Carriers for use with the present invention include permeable and semipermeable matrices or polymers that control the release characteristics of the formulation. Such polymers include, for example, cellulose acylates, acetates, and other semi-permeable polymers such as those described in U.S. Pat. No. 4,285,987 (hereby incorporated by reference), as well as the selectively permeable polymers formed by the coprecipitation of a polycation and a polyanioni as disclosed in U.S. Pat. Nos. 3,173,876; 3,276,586; 3,541,005; 3,541,006 and 3,546,142 (relevant portions incorporated herein by reference).

Other carriers for use with the present invention include, e.g., starch, modified starch, and starch derivatives, gums, including but not limited to xanthan gum, alginic acid, other alginates, benitoniite, veegum, agar, guar, locust bean gum, gum arabic, quince psyllium, flax seed, okra gum, arabinoglactin, pectin, tragacanth, scleroglucan, dextran, amylose, amylopectin, dextrin, etc., cross-linked polyvinylpyrrolidone, ion-exchange resins, such as potassium polymethacrylate, carrageenan (and derivatives), gum karaya, biosynthetic gum, etc. Other useful polymers include: polycarbonates (linear polyesters of carbonic acid); microporous materials (bisphenol, a microporous poly(vinylchloride), micro-porous polyamides, microporous modacrylic copolymers, microporous styrene-acrylic and its copolymers); porous polysulfones, halogenated poly(vinylidene), polychloroethers, acetal polymers, polyesters prepared by esterification of a dicarboxylic acid or anhydride with an alkylene polyol, poly(alkylenesulfides), phenolics, polyesters, asymmetric porous polymers, cross-linked olefin polymers, hydrophilic microporous homopolymers, copolymers or interpolymers having a reduced bulk density, and other similar materials, poly(urethane), cross-linked chain-extended poly(urethane), poly(imides), poly(benzimidazoles), collodion, regenerated proteins, semi-solid cross-linked poly(vinylpyrrolidone).

Additional additives and their levels, and selection of a primary coating material or materials will depend on the following properties: pH levels at target site, desirability to make tablet pH dependent or pH independent, solubility in alcohol, resistance to dissolution and disintegration in the stomach; impermeability to gastric fluids and drug/carrier/enzyme while in the stomach; ability to dissolve or disintegrate rapidly at the target intestine site; physical and chemical stability during storage; non-toxicity; easy application as a coating (substrate friendly); and economical practicality.

Further to the above, various formulations, not limiting the scope of the present invention, illustrating the invention are described hereafter. A controlled-release tablet or capsule or the like comprises colchicine as a core coated with an immediate release layer. A controlled-release double layer tablet or capsule or the like comprises a layer of sustained release and a layer of immediate release. A controlled-release tablet with more than two layers comprises (i) one or two more layers of substance controlling the sustained release and (ii) one or two more layers of immediate release.

According to one embodiment of the invention, the composition comprising colchicine salicylate may be further coated with at least one release-slowing intermediate layer of slightly soluble intermediate layer to control release of colchicine.

Traditionally, colchicine immediate release dosage forms (mostly tablets, also injections or oral solutions) have been used in the treatment of gout or FMF. Worldwide, all approved pharmaceuticals containing colchicine are approved only for gout and/or FMF and are immediate release tablets. Colchicine can be used in the prevention of certain other inflammatory diseases such as pericarditis, PPS and, most recently, patients with stable coronary heart diseases. The difference between treatment and prevention with regard to colchicine is that in treatment, an overt disease and/or ongoing inflammation has to be treated. Thus high levels of colchicine are required, which usually goes hand in hand with unwanted side effects, most prominently gastrointestinal insults. In prevention, one does not have to suppress ongoing inflammation but rather suppress an outbreak of inflammation. Thus, supposedly lower and steadier levels of colchicine are required and are beneficial. As described in the present invention, this is achieved by administering colchicine formulated as a sustained release preparation, as described above.

In the treatment of an acute inflammatory disease such as FMF or gout, high doses and high serum levels are necessary and desired to suppress inflammation. Therefore conventional tablets with a fast release and a rather low plasma half-life are suitable. However, in case of prevention of a cardiovascular disease, lower levels may be sufficient to inhibit neutrophil activity. The sustained release system facilitates more steady levels of colchicine and reduces the incidence of adverse events.

An advantage of colchicine administered as sustained release is, e.g., a flattening of the serum level curve (lower but broader peak levels) reduces the incidence of serious adverse events related to colchicine toxicity, also in case of potential drug interactions. Much of colchicine related toxicity comes from the fact that one or both of the excretion pathways (liver and kidney) is reduced in its activity, either by other drugs or by a disease (e.g. kidney insufficiency). In the case of a slower and extended drug absorption (extended release), the body has also more time to excrete the colchicine from the system. In this case, it is less likely that colchicine levels reach toxic levels in case of defective excretion (due to drug interaction or disease). In addition, administration of colchicine as sustained release is resistant to dose dumping, therefore the dissolution of the composition is not significantly influenced by alcohol.

Further, sustaining the release expands the time where colchicine is present in the blood in therapeutic levels. This results in a more efficient inhibition of disease progression and, thus, improving the clinical outcome.

Furthermore, for the prophylactic uses described herein, colchicine does not have to go deep into the tissue (like for gout), it may be active in the blood system directly (in the vessels) where it acts on the plaques and especially on inflammatory blood cells (neutrophils). This means, less total colchicine and lower serum levels can be therapeutic. Fast and high colchicine levels, e.g., as for treating an acute gout flare can be avoided. Thus, lower levels of colchicine, e.g., about 0.1 to about 0.75mg sustained release formulations as described above (or even less frequent doses), may be sufficient to achieve the desired clinical outcome.

In the normal situation, most colchicine is absorbed from the small intestine and most passes the liver (some is also excreted in the urine via kidney). There it is metabolized but quite a large proportion of colchicine goes through the liver un-metabolized. This means, it goes through the liver into the bile and from there it is excreted into the big intestine (colon). There it can be resorbed into the body again which leads to the characteristic second peak (accounts for about 50% of totally absorbed colchicine and is thought to be responsible for gastrointestinal problems, such as diarrhea). If colchicine salicylate is formulated as a sustained release preparation as described above, a slower release of colchicine results in a slower resorption. This results in more complete metabolism of colchicine or its derivative in the liver (because it is less busy with colchicine at a time) and, thus, less recirculation of un-metabolized colchicine. This consequently reduces the incidence of gastrointestinal problems. A colchicine derivative, i.e., colchicine salicylate, administered as sustained release in accordance with the present invention may also be beneficial for other known side/adverse effects associated with colchicine treatment/administration (the skilled person is well aware of the adverse effects that may occur upon colchicine administration or colchicine treatment). Thus, administration of a colchicine derivative, i.e., colchicine salicylate, as sustained release, as described above, results in a safety increase and safety benefit.

One of skill in this field can readily determine the pharmacokinetic parameters of colchicine salicylate, such as plasma concentration (C), Cmax, Cn, C24, Tmax, and AUC. In embodiments, colchicine salicylate exhibits an increase in Cmax compared to administration of colchicine free base or other colchicine salt forms. The increase can be from 2%, 5%, 6%, 7%, 8%, 9%, 10%, 11%, 12%, 13%, 14%, 16%, 17%, 18%, 19%, 20%, 21%, 22%, 23%, 24%, 25%, 26%, 27%, 28%, 29%, 30% or more. It would be beneficial to have such an increase in the maximum plasma concentration of colchicine. In embodiments, the AUC of colchicine salicylate exhibits an increase compared to that associated with the administration of colchicine free base or other colchicine salt forms. The increase can be from 2%, 5%, 6%, 7%, 8%, 9%, 10%, 11%, 12%, 13%, 14%, 16%, 17%, 18%, 19%, 20%, 21%, 22%, 23%, 24%, 25%, 26%, 27%, 28%, 29%, 30% or more. An increase in AUC for the same Cmax can correlate to an increased effect.

The bioavailability of colchicine is poor (ca. 45% with inter patient variations of 20-80%), which limits its efficacy in poor responders. An increase in bioavailability can provide a significant clinical benefit as it allows a reduction in the dose to achieve the same effect or, alternatively, a better effect can be achieved with the same dose. Without being bound to theory, salicylate may increase the lipophilicity of the entire molecule. This can increase the likelihood of uptake by GI cells, thereby increasing absorption (and bioavailability). Strong first pass elimination is also a factor believed to affect bioavailability. Therefore, in embodiments, the presently disclosed formulations of colchicine salicylate may provide the benefits of:
1. Administration of the same dose of colchicine as colchicine-salicylate and achieve a higher serum level and thereby potentially higher efficacy for certain indications; and/or
2. Administration of a reduced dose of colchicine as colchicine-salicylate to achieve the same dose as the free base or other salt forms.

With the increase in bioavailability, a reduced dosage of colchicine, in the form of colchicine-salicylate, is possible. For example, a 20% increase in bioavailability as described elsewhere herein can correspond to a 20% lower dose. Accordingly, in embodiments, the dosages and amounts of colchicine salicylate can be lowered in direct relation to the increase in bioavailability. Thus, in embodiments, the amount of colchicine salicylate in the formulations described herein is from about 0.1 mg to about 1.0 mg; from about 0.2 mg to about 0.7 mg; or from about 0.2 mg to about 0.675 mg. Useful values in that range include 0.20, 0.25, 0.30, 0.35, 0.40, 0.50, 0.55, 0.60, 0.65, and 0.675.

### IV. Methods of Preparing Colchicine Salicylate Formulations

The current invention additionally encompasses a method of preparing formulations comprising colchicine salicylate in both an immediate release and sustained release form.

In one embodiment, the colchicine salicylate compositions described in the present invention are in the form of a tablet. As used herein, the term "tablet" means a compressed pharmaceutical dosage form of any shape or size. The tablets described herein may be obtained from the compositions comprising colchicine and a pharmaceutically acceptable excipient. Any of the colchicine salicylate compositions can be in the form of any other dosage form known in the art, specifically, any oral dosage form, for example a capsule.

Colchicine salicylate may be prepared by, 1) contacting about 3 parts colchicine free base with about 1 part salicylic acid; and 2) adding an amount of water to prepare the salicylate salt. The salt can be dried to yield a powder. For example, colchicine salicylate may be prepared by mixing 20 mg of colchicine with 7 mg of salicylic acid, moistening the mixture with water, and then drying the mixture. The result is a yellow amorphous powder than can then be used in the preparation of a sustained release, delayed release or immediate release formulation.

In one embodiment of the invention, there is provided a sustained release formulation for use in oral dosage forms. The formulation includes a mixture containing hypromellose as a hydrophilic matrix, which is effective to provide sustained release of a pharmaceutically active ingredient.

Matrix systems are well known in the art. In a typical matrix system, the drug is homogenously dispersed in a polymer in association with conventional excipients. This admixture is typically compressed under pressure to produce a tablet. The API is released from the tablet by diffusion and erosion. Matrix systems are described in detail by (i) Handbook of Pharmaceutical Controlled Release Technology, Ed. D. L. Wise, Marcel Dekker, Inc. New York, N.Y. (2000), and (ii) Treatise on Controlled Drug Delivery, Fundamentals, Optimization, Applications, Ed. A. Kydonieus, Marcel Dekker, Inc. New York, N.Y. (1992), the contents of both of which are hereby incorporated by reference.

When the tablet is exposed to aqueous media, such as in the gastrointestinal tract, the tablet surface wets and the polymer begins to partially hydrate forming an outer gel layer. This outer gel layer becomes fully hydrated and begins to erode into the aqueous fluids. Water continues to permeate toward the core of the tablet permitting another gel layer to form beneath the dissolving outer gel layer. These successive concentric gel layers sustain uniform release of the API by diffusion from the gel layer and exposure through tablet erosion. In the case of the mixtures of the present invention, when included in a compressed tablet matrix, the hypromellose provides a hydrophilic swellable structure capable of functioning as the gel layer. In this way, the drug release is controlled.

In accordance with one embodiment, the colchicine salicylate composition of the present invention can be manufactured by either wet or dry granulation of a colchicine composition, blending the resulting granulate with excipients, and then compressing the composition into tablets.

In one embodiment, wet granulation is used to prepare wet granules comprising colchicine salicylate. A granulating liquid is used in wet granulation process. Both aqueous and non-aqueous liquids may be used as the granulating liquid. In one embodiment, the granulating liquid is an aqueous liquid, or more specifically, purified or de-ionized water. The amount of the granulating liquid used may depend on many factors, for example, the type of the granulating liquid, the amount of the granulating liquid used, the type of excipient used, the nature of the active agent, and the active agent loading.

In one embodiment, the colchicine particles and suitable excipients are mixed with the granulating liquid for a sufficiently long period to facilitate good distribution of all starting materials and good content uniformity. Wet granulation is generally performed at temperatures between about 20° C. to about 35° C., or more specifically, at room temperature (about 25° C.). Following wet granulation, the granulate is dried at increased temperatures to yield a dry granulate. In an embodiment, the step of drying may be performed for a sufficiently long period until the desired residual moisture content is reached. In an embodiment, this may be about 45°C for about 12-48 hours. It should be appreciated that the overall time to perform the granulation process may depend on a variety of factors, including but not limited to, the solvents used, batch size, instruments used, etc.

Any equipment may be used to contact the granulating liquid with the colchicine and the excipients as long as uniform distribution of the granulating liquid is achieved. For example, small-scale production can be achieved by mixing and wetting the colchicine and the excipients in mortars or stainless steel bowls, while for larger quantities, V-blenders with intensifier bars, planetary mixers, rotary granulators, high shear granulators, and fluid-bed granulation equipment may be used. In one embodiment, the granulator is a high shear granulator.

In one embodiment, a method of making a colchicine salicylate composition comprises wet granulating colchicine salicylate with pharmaceutically acceptable excipients and a granulating liquid to obtain wet granules, and mixing the granules in a next step with a second excipient to obtain a colchicine salicylate composition. In one embodiment, the pharmaceutically acceptable excipient comprises a binder and a filler. In an embodiment, the binder may be Hypromellose. In an embodiment, the filler may be lactose monohydrate and pregelatinized starch. In another embodiment, purified water is used as the granulating liquid. In an embodiment, the second excipient mixed with the granules may be a filler. In an embodiment, the filler may be lactose monohydrate. The colchicine salicylate compositions can contain about 0.1 wt % to about 10 wt %, or about 0.2 wt % to about 0.7 wt %, of colchicine salicylate, or more specifically, about 0.25 wt % to about 0.675 wt %, of colchicine salicylate, based on the total weight of the colchicine salicylate composition.

In one embodiment, a sustained release formulation of a colchicine salicylate composition comprises colchicine salicylate, a binder, a filler, a retarding agent, a glidant, and a lubricant. In an embodiment, a colchicine salicylate composition comprises about 0.5 to about 1.0 mg colchicine salicylate; about 10 to about 80 mg lactose monohydrate; about 5 to about 50 mg pregelatinized starch; about 10 to about 40 mg Hypromellose 6mPa^{∗}s; about 10 to about 30 mg lactose monohydrate; about 5 to about 40 mg Retalac (compound of lactose monohydrate and Hypromellose 4000mPa^{∗}s 50/50 w/w %); about 0.5 to about 5 mg Talc; and about 0.5 to about 5 mg Stearic acid 50. In an embodiment, the colchicine salicylate composition comprises about 0.675 mg colchicine salicylate, about 59 mg lactose monohydrate; about 7.5 mg pregelatinized starch; about 5mg lactose monohydrate; about 1 mg Hypromellose 6mPa^{∗}s; about 25 mg Retalac (compound of lactose monohydrate and Hypromellose 4000mPa^{∗}s 50/50 w/w %); about 1 mg Talc; and about 1 mg Stearic acid 50. The colchicine dosage form has a total weight of about 100 mg. The colchicine composition can be in the form of a tablet.

In another embodiment the invention, there is provided an immediate release formulation for use in oral dosage forms. The process for making the immediate release formulation is substantially the same as the one described above. In one embodiment, a method of making a colchicine salicylate composition comprises wet granulating colchicine salicylate with pharmaceutically acceptable excipients and a granulating liquid to obtain wet granules, and mixing the granules in a next step with a second excipient to obtain a colchicine salicylate composition. In one embodiment, the pharmaceutically acceptable excipient comprises a filler and a disintegrant. In an embodiment, the filler may be lactose monohydrate. In an embodiment, the disintegrant may be potato starch. In another embodiment, gelatine may be used as the granulating agent. The colchicine salicylate composition can contain about 0.1 wt % to about 10 wt %, or about 0.25 wt % to about 1.0 wt %, of colchicine salicylate, or more specifically, about 0.50 wt % to about 0.675 wt%, of colchicine salicylate, based on the total weight of the colchicine salicylate composition.

In one embodiment, an immediate release preparation of a colchicine salicylate composition comprises colchicine salicylate, a filler, a disintegrant, a granulation agent, a glidant, and a lubricant. In an embodiment, a colchicine salicylate composition comprises about 0.5 to about 1.0 mg colchicine salicylate; about 10 to about 95 mg lactose monohydrate; about 5 to about 50 mg potato starch; about 0.5 to about 5.0 mg gelatine; about 0.5 to about 5 mg Talc; and about 0.5 to about 5 mg Stearic acid 50. In an embodiment, the colchicine salicylate composition comprises about 0.675 mg colchicine salicylate, about 89 mg lactose monohydrate; about 7.325 mg potato starch; about 1.0 mg gelatine; about I mg Talc; and about 1 mg Stearic acid 50. The colchicine dosage form has a total weight of about 100 mg. The colchicine composition can be in the form of a tablet. In another embodiment, the colchicine salicylate composition comprises about 0.81 mg colchicine salicylate, about 89 mg lactose monohydrate; about 7.19 mg potato starch; about 1.0 mg gelatine; about 1 mg Talc; and about 1 mg Stearic acid 50. The colchicine dosage form has a total weight of about 100 mg. The colchicine composition can be in the form of a tablet.

In one embodiment, a tablet comprises: i. colchicine salicylate from about 0.1 wt% to about 1.0 wt%; ii. a filling agent from about 10 wt% to about 95 wt%; iii. a disintegrant from about 5 wt% to about 50 wt%; iv. a granulation agent from about 0.5 wt% to about 5.0 wt%; v. a lubricant from about 0.5 wt% to about 5.0 wt%; and vi.a glidant from about 0.5 wt% to about 5.0 wt%.

In one embodiment, a tablet comprises: i. colchicine salicylate from about 0.2 wt% to about 0.7 wt%; ii. a filling agent from about 10 wt% to about 95 wt%; iii. a disintegrant from about 5 wt% to about 50 wt%; iv. a granulation agent from about 0.5 wt% to about 5.0 wt%; v. a lubricant from about 0.5 wt% to about 5.0 wt%; and vi.a glidant from about 0.5 wt% to about 5.0 wt%.

In one embodiment, a tablet comprises: i. colchicine salicylate from about 0.5 wt% to about 1.0 wt%; ii. a filling agent from about 10 wt% to about 95 wt%; iii. a disintegrant from about 5 wt% to about 50 wt%; iv. a granulation agent from about 0.5 wt% to about 5.0 wt%; v. a lubricant from about 0.5 wt% to about 5.0 wt%; and vi.a glidant from about 0.5 wt% to about 5.0 wt%.

In one embodiment, a tablet comprises: i. colchicine salicylate from about 0.6 wt% to about 0.9 wt%; ii. a filling agent from about 85 wt% to about 95 wt%; iii. a disintegrant from about 5 wt% to about 10 wt%; iv. a granulation agent from about 0.5 wt% to about 5.0 wt%; v. a lubricant from about 0.5 wt% to about 1.5 wt%; and vi. a glidant from about 0.5 wt% to about 1.5 wt%.

In one embodiment, a tablet comprises: i. colchicine salicylate at about 0.675 wt%; ii. a filling agent at about 89 wt%; iii. a disintegrant from about 6.9 wt% to about 7.5 wt%; iv.a granulation agent at about 1.0 wt%; v. a lubricant at about 1.0 wt%%; and vi. a glidant at about 1.0 wt%.

In one embodiment, a tablet comprises: i. colchicine salicylate at about 0.675 wt%; ii. lactose monohydrate at about 89 wt%; iii. potato starch from about 6.9 wt% to about 7.5 wt%; iv. gelatin at about 1.0 wt%; v. stearic acid at about 1.0 wt%%; and vi. talc at about 1.0 wt%.

In all embodiments, the tablet can exhibit an increase in bioavailability of said colchicine salicylate.

In another embodiment, a tablet comprises: colchicine salicylate from about 0.1 wt% to about 1.0 wt%; ii. a filling agent(s) from about 10 wt% to about 95 wt%; iii. a binder from about 0.1 wt% to about 2.0%; iv. a retarding agent from about 5.0 wt% to about 40.0 wt%; v. a lubricant from about 0.5 wt% to about 5.0 wt%; and vi. a glidant from about 0.5 wt% to about 5.0 wt%; wherein the tablet exhibits an increase in bioavailability of colchicine salicylate and wherein the tablet exhibits sustained release of colchicine salicylate.

In another embodiment, a tablet comprises: colchicine salicylate from about 0.2 wt% to about 0.7 wt%; ii. a filling agent(s) from about 10 wt% to about 95 wt%; iii. a binder from about 0.1 wt% to about 2.0 wt%; iv. a retarding agent from about 5.0 wt% to about 40.0 wt%; v. a lubricant from about 0.5 wt% to about 5.0 wt%; and vi. a glidant from about 0.5 wt% to about 5.0 wt%; wherein the tablet exhibits an increase in bioavailability of colchicine salicylate and wherein the tablet exhibits sustained release of colchicine salicylate.

In another embodiment, a tablet comprises: colchicine salicylate from about 0.5 wt% to about 1.0 wt%; ii. a filling agent(s) from about 10 wt% to about 95 wt%; iii. a binder from about 0.1 wt% to about 2.0 wt%; iv. a retarding agent from about 5.0 wt% to about 40.0 wt%; v. a lubricant from about 0.5 wt% to about 5.0 wt%; and vi. a glidant from about 0.5 wt% to about 5.0 wt%; wherein the tablet exhibits an increase in bioavailability of colchicine salicylate and wherein the tablet exhibits sustained release of colchicine salicylate.

In another embodiment, a tablet comprises: colchicine salicylate at about 0.675 wt%; ii. a filling agent(s) at about 70 wt%; iii. a binder at about 1.0 wt%; iv. a retarding agent at about 25.0 wt%; v. a lubricant at about 1.0 wt%; and vi. a glidant at about 1.0 wt%. wherein the tablet exhibits an increase in bioavailability of colchicine salicylate and wherein the tablet exhibits sustained release of colchicine salicylate.

In yet another embodiment, a tablet comprises i. colchicine salicylate at about 0.675 wt%; ii. lactose monohydrate at about 63 wt%; iii. pregelatinized starch at about 7.5 wt%; iv. hypromellose at about 1.0 wt%; iv. Retalac at about 25.0 wt%; v. stearic acid 50 at about 1.0 wt%; and vi. talc at about 1.0 wt%, wherein the tablet exhibits an increase in bioavailability of colchicine salicylate and wherein the tablet exhibits sustained release of colchicine salicylate.

In an embodiment, the method of making a composition comprises wet granulating colchicine salicylate with a pharmaceutically acceptable excipient to obtain wet granules, and mixing the granules with a filler to obtain a colchicine salicylate composition. In some embodiments, the method further includes drying the mixture. In another embodiment, the wet granules are dried to obtain dried granules, and then the dried granules are mixed with a binder, a filler, or both to obtain the composition. In another embodiment, the dried granules can be milled to obtain milled granules before mixing the milled dried granules with the binder, a filler, or both. The method can further include mixing the colchicine salicylate composition with a glidant, a lubricant, or both to obtain a blend or compressing the blend to obtain a tablet. In one embodiment, the glidant may be Talc. In another embodiment, the lubricant may be Stearic acid. The method can further include coating the tablet.

In another embodiment, a method of making a colchicine salicylate tablet comprises wet granulating colchicine salicylate with a pharmaceutically acceptable excipient to obtain wet granules; drying the wet granules to obtain dried granules; milling the dried granules to obtain milled granules; mixing the milled granules with a filler to obtain the composition; mixing the composition with a glidant, a lubricant, or both to obtain a blend; and compressing the blend to obtain a colchicine tablet of the present invention.

In some embodiments, the wet granules are dried to obtain dried granules before mixing with a second excipient, for example a filler. Wet granules can be dried by any suitable means to remove the granulating liquid and to form dried granules containing colchicine and the pharmaceutically acceptable excipient. The conditions and duration of drying depend on factors such as the liquid used and the weight of the granulating particles. Examples of suitable drying methods include, but are not limited to, tray drying, forced air drying, microwave drying, vacuum drying and fluid bed drying.

After drying, dried granules may be mixed directly with an excipient, for example, a filler, a binder, or a lubricant, for further processing. Alternatively, dried granules may optionally be subjected to additional processing steps prior to mixing with the excipient. For example, dried granules may be sized to reduce particle size prior to mixing with an excipient. Exemplary sizing operations include milling or sieving. Any suitable equipment for reducing the particle size may be used in the present invention.

Suitable excipients may be added extragranularly and mixed with the granules to form colchicine compositions. As used herein, the term "extragranular" or "extragranularly" means that the referenced material, for example, a suitable excipient, is added or has been added as a dry component after wet granulation. In one embodiment, a filler, a binder, a glidant and a lubricant are added extragranularly to the granules and mixed to form a blend. The blend may be encapsulated directly into capsule shells, for example, hard gelatin shells, to form capsule formulations. Alternatively, the blend may be compressed into tablets. In some embodiments, the granules are dried granules or milled, dried granules.

Mixing can be carried out for a sufficient time to produce homogeneous mixtures or blends. Mixing may be accomplished by blending, stiffing, shaking, tumbling, rolling, or by any other method to achieve a homogeneous blend. In some embodiments, the components to be mixed are combined under low shear conditions in a suitable apparatus, such as a V-blender, tote blender, double cone blender or any other apparatus capable of functioning under low shear conditions.

The homogenous mixtures or blends are then compressed using any method suitable in the industry. The mechanical force will define the physical properties of the tablets, especially the crushing strength of the resulting tablet. The mechanical strength interacts with the initial swelling of the tablet and dilution speed of the tablet core. This effect is well known in the art and can be adjusted and controlled during the lifecycle of the product. For the colchicine sustained-release formulation of the present invention, the compression strengths used may range from about 30N to about 130N. In one embodiment, the compression strength may be about 100N. In another embodiment, the compression strength may be about 100N +/- 15N.

The colchicine salicylate tablets prepared from the above described methods exhibit acceptable physical characteristics including good friability and hardness. As per EP and USP guidelines, the colchicine tablets disclosed herein have friability in the range of about 0% to less than about 1%.

The colchicine salicylate tablet can be coated. Coating the tablet may be performed by any known process. A coating for the tablet disclosed herein can be any suitable coating, such as, for example, a functional or a non-functional coating, or multiple functional or non-functional coatings. By "functional coating" is meant to include a coating that modifies the release properties of the total formulation, for example, a sustained-release coating. By "non-functional coating" is meant to include a coating that is not a functional coating, for example, a cosmetic coating. A non-functional coating can have some impact on the release of the active agent due to the initial dissolution, hydration, perforation of the coating, etc., but would not be considered to be a significant deviation from the non-coated composition.

### V. Treatment Methods Using Colchicine Derivatives

The present invention also presents a method of treatment or prevention of cardiovascular diseases and/or inflammatory disorders in a subject, comprising administering to the subject a therapeutically effective amount of colchicine salicylate of the present invention.

In one embodiment, treatment includes the application or administration of a colchicine salicylate formulation as described herein to a patient, where the patient has, or has the risk of developing a cardiovascular disease and/or an inflammatory disorder.

As used herein, the term "cardiovascular disease" refers to any disease involving the heart and/or the vascular system (all blood vessels incl. arteries, capillaries and veins). This includes all diseases listed in chapter IX "Diseases of the circulatory system (I00-I99)" of the International Statistical Classification of Diseases and Related Health Problems 10th Revision (ICD-10) Version for 2010, by the World Health Organisation.

More specifically, all diseases of this ICD-10 class which involve a) inflammation of any part of the heart or blood vessel as well as b) ischemia/atherosclerosis/thickening of any blood vessel of the circulatory system.

Colchicine as described herein is for use in the treatment and/or prevention of any inflammatory disorder involving the heart tissue selected from ICD-10 sections I00-I99. More specifically, these include acute and recurrent pericarditis as well as post-surgical complications involving inflammation of the pericardium (Postcardiotomy syndrome, Postpericardiotomy syndrome, pericardial effusion). Other inflammatory heart diseases covered are any form of myocarditis, endocarditis and arterial fibrillation.

The proposed mechanism of action in this indication is the inhibition of plaque instability by neutrophil inhibition. In stable coronary disease, fatty materials accumulate at the blood vessel and form a stable plaque. This plaque may become subject to attack by neutrophils. This may cause plaque instability and consequently leads to plaque rupture and clinical events. Therefore, colchicine as described herein is for use in treatment and/or prevention of any disease of the cardiovascular system which involves ischemia, atherosclerosis and/or thickening of blood vessels (arteries, capillaries, veins) due to plaque formation with a risk of clinical events due to plaque instability. Claimed are all diseases classified in ICD-10 section I00-I99 fulfilling any of these requirements. Examples are stable coronary disease, cardiovascular atherosclerosis, and atherosclerosis of the peripheral vascular system, Abdominal Aortic Aneurysm (AAA) and carotid and iliofemoral/renal atheromas (e.g. 125, 170).

In the context of the present invention, an acute cardiovascular event in a patient with stable cardiovascular disease is preferably a cardiovascular event in a patient with stable coronary disease. In the context of the present invention, the term "stable coronary disease" and "stable coronary heart disease" have the same meaning and are used interchangeable. Both terms include the medical condition stable coronary artery disease (SCAD). "Stable" in the context of the terms "stable cardiovascular disease", "stable coronary disease" or "stable coronary heart disease" is defined as any conditions of diagnosed cardiovascular disease in the absence of acute cardiovascular events. Hence, e.g. stable coronary disease defines the different evolutionary phases of coronary disease, excluding the situations in, which coronary artery thrombosis dominates clinical presentation (acute coronary syndrome).

A colchicine salicylate composition of the present invention can be used for the prevention and/or treatment of cardiovascular diseases. Cardiovascular diseases include, but are not limited to, heart diseases as described, e.g., in Robbins and Cotran, Pathologic Basis of Disease, Eighth Edition, Saunders Elsevier. Cardiovascular disease refers to a group of diseases of the circulatory system including the heart, blood and lymphatic vessels. In particular, cardiovascular disease may include vascular diseases involving atherosclerosis, plaque formation or disposition. The most common cardiovascular diseases are coronary heart disease and stroke. Non-limiting examples of cardiovascular disease which may be prevented or treated according to the methods of the invention include coronary heart disease (disease of the blood vessels supplying the heart muscle), cerebrovascular disease (disease of the blood vessels supplying the brain), peripheral arterial disease (disease of blood vessels supplying the arms and legs), rheumatic heart disease (damage to the heart muscle and heart valves from rheumatic fever, caused by streptococcal bacteria), congenital heart disease (malformations of heart structure existing at birth), deep vein thrombosis and pulmonary embolism (blood clots in the leg veins, which can dislodge and move to the heart and lungs), hyperlipemia (an excessive level of blood fats, such as LDL), high blood pressure, coronary artery disease, atherosclerosis, ischemic diseases, abdominal aortic aneurism, carotid and iliofemoral/renal atheromas, heart failure, cardiac rhythm defects, arteriosclerosis, heart attack and stroke. Heart attacks and strokes are usually acute events and are mainly caused by a blockage that prevents blood from flowing to the heart or brain. The most common reason for this is a build-up of fatty deposits on the inner walls of the blood vessels that supply the heart or brain. Strokes can also be caused by bleeding from a blood vessel in the brain or from blood clots.

Especially, in the context of the present invention, a composition comprising colchicine salicylate can be used for the prevention of acute pericarditis, recurrent pericarditis, recurrent pericarditis in patients with a history of pericarditis, post-pericardiotomy syndrome (PPS), PPS in patients undergoing cardiac surgery, and cardiovascular events in patients with stable coronary (heart) disease (the cardiovascular events can be acute cardiovascular events).

Pericarditis is an inflammatory disease involving the pericardium, a thin double-walled fibroelastic sac, surrounding the heart. Due to inflammation, it comes to irritation and swelling of the pericardium. This causes the sac to rub against the heart which causes chest pain, the most common symptom of pericarditis. Pericarditis is the most common form if inflammatory disorder of the heart, though very rare on a population basis. Pericarditis is very heterogeneous in its origin, clinical manifestations and duration of symptoms. It can either occur as isolated clinical problem or as a manifestation of a systemic disease. In most cases (90%), pericarditis is of idiopathic (spontaneous, unknown) etiology but may also occur secondary to systemic infections, acute myocardial infarction or autoimmune diseases. The post-pericardiotomy syndrome (PPS) may be a troublesome complication following cardiac surgery occurring a few days to several weeks after the surgical operation. The estimated incidence of the syndrome has a relatively wide range affecting from 10 to 40% of patients submitted to cardiac surgery (Prince and Cunhe, 1997, Heart Lung, 26:165).

Non-limiting examples of (acute) cardiovascular events are injury of the atherosclerotic wall, acute coronary syndrome, out-of-hospital cardiac arrest, or non-cardioembolic ischemic stroke. Further such events are described, e.g., in Robbins and Cotran, Pathologic Basis of Disease, Eighth Edition, Saunders Elsevier.

In an embodiment, the colchicine salicylate formulation of the present invention may be used to treat inflammatory disease other than those mentioned above. In an embodiment, the inflammatory disease includes, but is not limited to, gout, familial Mediterranean fever, Behcet's disease, Age-related macular degeneration and Alzheimer's disease.

Patients/subjects which suffer from the above described disease and/or which are suitable for treatment with colchicine according to the present invention can be diagnosed by conventional and/or routine procedures. The skilled person is well aware of them. Diagnosis is also described, e.g., in Robbins and Cotran, Pathologic Basis of Disease, Eighth Edition, Saunders Elsevier.

In an embodiment, colchicine salicylate as described herein is useful for the treatment of various cardiovascular diseases and/or inflammatory disorders. In some embodiments, treatment of a cardiovascular disease and/or an inflammatory disorder is intended to include remediation of, improvement of, amelioration of, lessening of the severity of, or reduction in the time course of, a disease, disorder or condition, or any parameter or symptom thereof. In an embodiment of the present invention, the term "disorder", "disease", or "condition" is to be understood as cardiovascular disease and/or inflammatory disorder as described above. In the context of the present invention, "amelioration" refers, without limitation, to any observable beneficial effect.

In accordance with the present invention, colchicine salicylate as described herein can be used to promote a positive therapeutic response with respect to the cardiovascular disease and/or inflammatory disorder. A "positive therapeutic response" with respect to the cardiovascular disease and/or inflammatory disorder is intended to include an improvement in the disease can be evidenced by, for example, a delayed onset of clinical symptoms of the disease or condition, a reduction in severity of some or all clinical symptoms of the disease or condition, a slower progression of the disease or condition, an improvement in the overall health or well-being of the subject, or by other parameters well known in the art that are specific to the particular disease.

In another embodiment, colchicine salicylate as described herein is useful in the prevention of various cardiovascular diseases and/or inflammatory disorders. In the context of the present invention, the term "prevention" is well known in the art. For example, a patient/subject suspected of being prone to suffer from a disorder or disease as defined herein may, in particular, benefit from a prevention of the disorder or disease. The subject/patient may have a susceptibility or predisposition for a disorder or disease, including but not limited to hereditary predisposition. Such a predisposition can be determined by standard assays, using, for example, genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of compounds of the present invention before any clinical and/or pathological symptoms are diagnosed or determined or can be diagnosed or determined by the attending physician. Prevention includes, without limitation, to avoid the disease or condition from occurring in patient and/or subject that may be predisposed to the disease but does not yet experience or exhibit symptoms of the disease (prophylactic treatment).

A composition comprising colchicine salicylate according to the present invention may also be used in combination with conventional therapy for any of the diseases disclosed herein. Such conventional therapies are well known in the art and the skilled person knows any such therapies. A composition comprising colchicine salicylate as described herein may also be used in combination with colchicine-compatible statins. In general, non-limiting examples of statins are atorvastatin (Lipitor^{®}, Torvast^{®}), fluvastatin (Lescol^{®}), lovastatin (Mevacor^{®}, Altocor^{®}, Altoprev^{®}), pitavastatin (Livalo^{®}, Pitava^{®}), pravastatin (Pravachol^{®}, Selektine^{®}, Lipostat^{®}), rosuvastatin (Crestor^{®}) and simvastatin (Zocor^{®}, Lipex^{®}). In connection with the present invention, colchicine-compatible statins are used. Preferably, in the context of the present invention, the combination of the composition of the present invention with the colchicine-compatible statins are accomplished in connection with stable coronary heart disease. Such statins preferably are statins which, due to the nature of their mechanism of action, metabolism and/or clearance do not, or only to a small extent, interfere with the mechanism of action, metabolism and/or clearance of colchicine and therefore show a reduced risk, severity and/or incidence of drug-related drug adverse events when given in combination with colchicine. As described in U.S. Application Nos. 14/603,049 and 14/603,085, filed January 22, 2015, each of which is herein incorporated by reference in its entirety, the colchicine salicylate formulations disclosed herein may be administered to a subject in need thereof with antiplatelet agents and/or antithrombotic agents.

In an embodiment, the use of a composition comprising is in fixed (within the same pharmaceutical preparation) or unfixed (different pharmaceutical preparation) combination. "Fixed combination" is to be understood as meaning a combination whose active ingredients are combined at fixed doses in the same vehicle (single formula) that delivers them together to the point of application. Fixed combination can mean, e.g., in a single tablet, solution, cream, capsule, gel, ointment, salve, patch, suppository or transdermal delivery system. "Unfixed combination" as used herein is to be understood as meaning that the active ingredients/components are in more than one vehicle (e.g. tablets, solutions, creams, capsules, gels, ointments, salves, patches, suppositories or transdermal delivery systems). Each of the vehicles can contain a desired pharmaceutical composition or active component. For example, a preferred unfixed combination as described herein means that one vehicle contains colchicine, as described herein, and another vehicle contains a colchicine-compatible statin, as described herein. Examples of colchicine as described herein in fixed or unfixed combination(s) encompass(es) colchicine in combination with one or more colchicine-compatible statins selected from the group consisting of atorvastatin, rosuvastatin, simvastatin and pravastatin. Specifically, colchicine salicylate as described herein in fixed or unfixed combination is to be understood as meaning colchicine in combination with atorvastatin. Specifically, colchicine salicylate as described herein in fixed or unfixed combination is to be understood as meaning a composition comprising colchicine salicylate in combination with rosuvastatin. Specifically, colchicine salicylate as described herein in fixed or unfixed combination is to be understood as meaning colchicine in combination with simvastatin. Specifically, colchicine salicylate as described herein in fixed or unfixed combination is to be understood as meaning colchicine in combination with pravastatin.

In some embodiments, colchicine salicylate as described herein may be used in combination with a statin and another agent, such as ezetimibe/simvastatin. Colchicine salicylate as described herein may be used in combination with other drugs, e.g, which are used in medicine and are known to the skilled person (e.g. antibiotics, NSAID (non-steroidal anti-inflammatory drugs), corticosteroids).

### VI. Administration Methods

Methods of administering colchicine salicylate of the present invention to a subject in need thereof are well known to or are readily determined by those skilled in the art. The route of administration of the colchicine formulation can be, for example, oral, parenteral, by inhalation or topical. The term parenteral as used herein includes, e.g., intravenous, intraarterial, intraperitoneal, intramuscular, subcutaneous, rectal, or vaginal administration.

Colchicine salicylate when used as a composition in the context of the present invention may include one or more pharmaceutically acceptable carriers and thus may be prepared in the form of a local formulation, in order for it to be administered. The pharmaceutically acceptable carrier may include saline, sterile water, linger liquid, buffer saline, a dextrose solution, a malto dextrin solution, glycerol, ethanol and mixtures of one or more thereof, and also may include an additive such as an antioxidant, a buffer, a bacteriostatic agent or the like, as necessary. Furthermore, a diluent, a dispersant, a surfactant, a binder and a lubricant may be added when the composition according to the present invention is prepared, e.g., in the form of a local formulation such as an ointment, lotion, cream, gel, skin emulsion, skin suspension, patch or spray.

Non-limiting examples for administration of the compound and or compositions according to the present invention include coated and uncoated tablets, soft gelatine capsules, hard gelatine capsules, lozenges, troches, solutions, emulsions, suspensions, syrups, elixiers, powders and granules for reconstitution, dispersible powders and granules, medicated gums, chewing tablets and effervescent tablets. The composition according to the present invention can be administered in any pharmaceutical form for oral (e.g. solid, semi-solid, liquid), dermal (e.g. dermal patch), sublingual, parenteral (e.g. injection), ophthalmic (e.g. eye drops, gel or ointment) or rectal (e.g. suppository) administration. In an embodiment, the composition is formulated as a tablet, capsule, suppository, dermal patch or sublingual formulation.

The pharmaceutical compositions used in this invention comprise pharmaceutically acceptable carriers, including, *e.g*., ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol, and wool fat.

Certain pharmaceutical compositions used in this invention can be orally administered in an acceptable dosage form including, *e.g*., capsules, tablets, aqueous suspensions or solutions. Certain pharmaceutical compositions also can be administered by nasal aerosol or inhalation. Such compositions can be prepared as solutions in saline, employing benzyl alcohol or other suitable preservatives, absorption promoters to enhance bioavailability, and/or other conventional solubilizing or dispersing agents.

The amount of colchicine salicylate to be combined with the carrier materials to produce a single dosage form will vary depending upon the host treated and the particular mode of administration. The composition can be administered as a single dose, multiple doses or over an established period of time in an infusion. Dosage regimens also can be adjusted to provide the optimum desired response (*e.g*., a therapeutic or prophylactic response).

In some situations, the composition of the present invention can be parenterally administered. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include, *e.g*., water, alcoholic/aqueous solutions, emulsions or suspensions, including saline and buffered media. In the subject invention, pharmaceutically acceptable carriers include, but are not limited to, 0.01-0.1 M and preferably 0.05 M phosphate buffer or 0.8% saline. Other common parenteral vehicles include sodium phosphate solutions, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present such as, for example, antimicrobials, antioxidants, chelating agents, and inert gases and the like.

Parenteral formulations can be a single bolus dose, an infusion or a loading bolus dose followed with a maintenance dose. These compositions can be administered at specific fixed or variable intervals, *e.g*., once a day, or on an "as needed" basis.

The composition according to the present invention may be administered in a dose range varying depending on the patient's body weight, age, gender, health condition, diet, administration time, administration method, excretion rate and disease severity. The compounds of the present invention as compounds *per se* in their use as pharmacophores or as pharmaceutical compositions can be administered to the patient and/or subject at a suitable dose. The dosage regiment will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Generally, the regimen as a regular administration of the pharmaceutical composition comprising the herein defined should be, e.g., in a range as described below. Progress can be monitored by periodic assessment.

The composition according to the present invention can be administered with a single dose or with 2, 3, 4, 5, 6, 7, 8, 9, or 10 doses, if desired. The composition can be administered 1, 2, 3, 4, 5, 6, 7, 8, 9 or 10 times per day. Preferably, colchicine according to the present invention is administered once per day. More preferably, colchicine according to the present invention is administered once per day as a single dose.

The composition according to the present invention can be administered regularly for long periods of time. In an embodiment, the composition can be administered regularly for 1, 2, 3, 4, 5, 6, 7, 8, 9, 10 or more years. In another embodiment, the composition can be administered regularly for 1, 2, 3, 4, 5, 6, 7, 8, 9, 10, 11, 12 or more months. In other embodiments, the composition can be administered regularly for 1, 2, 3, 4, 5, 6, 7, 8, 9, 10 or more weeks. As used herein, the term "regularly" refers to administration of the composition at regular times or intervals over a period of time. For instance, the composition may be administered to a patient once daily for three years. In other embodiments, the composition may be administered to a patient once every other day for 5 years. It should be appreciated that the frequency of administration may vary based on a number of factors, including, but not limited to, the severity of disease, the overall health of the patient, any additional medications the patient is taking, and whether the treatment is prophylactic or not. It should also be appreciated that the frequency of administration may be adjusted at any point.

The amount/concentration/dose of the composition according to the present invention can be between 0.1 mg and 5.0mg, 0.1 mg and 2.0mg, 0.1 mg to 1.5mg, 0.1mg to 1.0mg, 0.1mg to 0.75mg, 0.1mg to 0.5mg, 0.25mg to 5.0mg, 0.25mg to 2.0mg, 0.25mg to 1.5mg, 0.25mg to 1.0mg, 0.25mg to 0.75mg, 0.25mg to 0.675mg, or 0.25mg to 0.5mg. In an embodiment, the composition according to the present invention is administered at a daily dose of colchicine salicylate of between about 0.1mg and about 0.75mg or between about 0.1mg and about 0.675mg. In another embodiment, the composition according to the present invention is administered at a daily dose of colchicine salicylate of between about 0.25mg to about 0.75mg or between about 0.25mg to about 0.675mg. In an embodiment, the composition according to the present invention is administered at a daily dose of about 0.75mg colchicine salicylate. In an embodiment, the composition according to the present invention is administered at a daily dose of about 0.675mg colchicine salicylate.

In a preferred embodiment, the amount/concentration of colchicine as used herein can be administered at the first day of administration in a higher dose (concentration/amount) compared to the administration of colchicine at the following days(s) of administration (maintenance administration/maintenance dose of administration). Alternatively such decreased dose (maintenance dose) can be started after 2, 3, 4, 5, 6, 7, 8, 9 or 10 days of initial administration of the higher dose. In case the course of treatment is any such as described above, the higher dose/amount/concentration of colchicine salicylate (e.g. at the first day of administration) can be any as described above, provided that the maintenance dose (the dose/amount/concentration of colchicine salicylate at the days following the higher dose/amount/concentration) is lower than the initial dose/amount/concentration of colchicine salicylate (e.g. at the first day of administration). Preferably, the composition of the invention is administered with a dose of colchicine salicylate of between about 1.0 mg to about 2.0 mg at the first day (preferably as a single dose) of administration and the maintenance dose of colchicine salicylate at the following day(s) of administration is between about 0.5 mg to about 1.0 mg. Preferably, the composition of the invention is administered with a dose of colchicine salicylate of between about 0.5 mg to about 1.0 mg at the first day (preferably as a single dose) of administration and the maintenance dose of colchicine salicylate at the following day(s) of administration is between about 0.25 mg to about 0.5 mg.

In keeping with the scope of the present disclosure, colchicine salicylate of the present invention can be administered to a human or other animal in accordance with the aforementioned methods of treatment in an amount sufficient to produce a therapeutic effect. Colchicine salicylate can be administered to such human or other animal in a conventional dosage form prepared by combining colchicine salicylate of the present invention with a conventional pharmaceutically acceptable carrier or diluent according to known techniques. It will be recognized by one of skill in the art that the form and character of the pharmaceutically acceptable carrier or diluent is dictated by the amount of active ingredient with which it is to be combined, the route of administration and other well-known variables.

By "therapeutically effective dose or amount" or "effective amount" is intended an amount of colchicine salicylate that when administered brings about a positive therapeutic response with respect to treatment of a patient with a disease to be treated, e.g., an improvement in the disease can be evidenced by, for example, a delayed onset of clinical symptoms of the disease or condition, a reduction in severity of some or all clinical symptoms of the disease or condition, a slower progression of the disease or condition, an improvement in the overall health or well-being of the subject, or by other parameters well known in the art that are specific to the particular disease.

The invention also provides for the use of colchicine salicylate in the manufacture of a medicament for treating a subject for treating a cardiovascular disease and/or inflammatory disorder, wherein the medicament is used in a subject that has been pretreated or is concurrently being treated with at least one other therapy. By "pretreated" or "pretreatment" is intended the subject has received one or more other therapies prior to receiving the medicament comprising the colchicine formulation. "Pretreated" or "pretreatment" includes subjects that have been treated with at least one other therapy within 2 years, within 18 months, within 1 year, within 6 months, within 2 months, within 6 weeks, within 1 month, within 4 weeks, within 3 weeks, within 2 weeks, within 1 week, within 6 days, within 5 days, within 4 days, within 3 days, within 2 days, or even within 1 day prior to initiation of treatment with the medicament comprising the colchicine formulation. By "concurrent" or "concomitant" is intended the subject is receiving one or more other therapies while at the same time receiving the medicament comprising the colchicine derivative, i.e., colchicine salicylate. It is not necessary that the subject was a responder to pretreatment with the prior therapy or therapies or a responder to the concurrent therapy or therapies. Thus, the subject that receives the medicament comprising colchicine salicylate could have responded, or could have failed to respond, to pretreatment with the prior therapy, or to one or more of the prior therapies where pretreatment comprised multiple therapies.

All of the references cited above, as well as all references cited herein, are incorporated herein by reference in their entireties.

While the invention has been illustrated and described in detail in above, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

The present invention is additionally described by way of the following illustrative non-limiting examples that provide a better understanding of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLES

### Example 1: Colchicine-salicylate Immediate Release Tablet

These formulations illustrate a colchicine-salicylate immediate release tablet. The tablet uses the ingredients and concentrations shown in Table 1 below.

| **Ingredient** | **mg / Tablet** | **Tablet %** | **Function** |
|---|---|---|---|
| Colchicine-salicylate | 0.675* | 0.675 | Active Pharmaceutical Ingredient (API) |
| Lactose monohydrate | 89.000 | 89.000 | Filling Agent |
| Potato starch | 7.325 | 7.325 | Disintegrant |
| Gelatine | 1.000 | 1.000 | Granulation Agent |
| Stearic acid | 1.000 | 1.000 | Lubricant |
| Talc | 1.000 | 1.000 | Glidant |
| Purified water** | q.s | - | Diluent for API and Binder |
| ***Total tablet weight [mg]:*** | **100.00** | **100.00** | |

| | | | |
|---|---|---|---|
| * equals approx. 0.500 mg colchicine ** used as granulating fluid, present in finished product with less than 4% | | | |

| **Ingredient** | **mg / Tablet** | **Tablet %** | **Function** |
|---|---|---|---|
| Colchicine-salicylate | 0.81 | 0.81 | Active Pharmaceutical Ingredient (API) |
| Lactose monohydrate | 89.000 | 89.000 | Filling Agent |
| Potato starch | 7.19 | 7.19 | Disintegrant |
| Gelatine | 1.000 | 1.000 | Granulation Agent |
| Stearic acid | 1.000 | 1.000 | Lubricant |
| Talc | 1.000 | 1.000 | Glidant |
| Purified water** | q.s | - | Diluent for API and Binder |
| ***Total tablet weight [mg]:*** | **100.00** | **100.00** | |

| | | | |
|---|---|---|---|
| * equals approx. 0.60 mg colchicine ** used as granulating fluid, present in finished product with less than 4% | | | |

The concentrations may be altered to change certain properties of the formulations, for instance, the dissolution profile. Table 2 shows the ranges for each ingredient.

| **Ingredient** | **Range** |
|---|---|
| Colchicine-salicylate | 0.5-1.0 % |
| Lactose monohydrate | 10-95 % |
| Potato starch | 5-50 % |
| Gelatine | 0.5-5.0 % |
| Stearic acid | 0.5-5.0 % |
| Talc | 0.5-5.0 % |
| Purified water** | q.s |
| ***Total tablet weight*** | **50-200 mg** |

### Example 2: Colchicine-salicylate Immediate Release Tablet

Colchicine-salicylate tablets may also be obtained by complexation of colchicine and salicylic acid during the manufacturing process of the tablets (by dissolution of colchicine and salicylic acid in an appropriate medium and stirring for sufficient time). This is done by adding colchicine and salicylic acid in a ratio of approx. 20 parts colchicine and approx. 7 parts salicylic acid (about 74.4% colchicine and about 25.6% salicylic acid) into the manufacturing process, yielding 100% Colchicine-salicylate. The following formulations illustrate a colchicine-salicylate immediate release tablet. The tablet uses the ingredients and concentrations shown in Table 3 below.

| **Ingredient** | **Ingredients in mg per 100mg** | **Composition in mg/Tablet** |
|---|---|---|
| Colchicine* | 0.500 | - |
| Salicylic acid* | 0.175 | - |
| Colchicine-salicylate | - | 0.675 |
| Lactose monohydrate | 89.000 | 89.000 |
| Potato starch | 7.325 | 7.325 |
| Gelatine | 1.000 | 1.000 |
| Stearic acid | 1.000 | 1.000 |
| Talc | 1.000 | 1.000 |
| Purified water** | q.s | - |
| ***Total tablet weight [mg]:*** | **100.00** | **100.00** |

| | | |
|---|---|---|
| * free colchicine and salicylic acid not present in finished product in significant amounts ** used as granulating fluid, present in finished product with less than 4% | | |

| **Ingredient** | **Ingredients in mg per 100mg** | **Composition in mg/Tablet** |
|---|---|---|
| Colchicine* | 0.60 | - |
| Salicylic acid* | 0.21 mg | - |
| Colchicine-salicylate | - | 0.81 |
| Lactose monohydrate | 89.000 | 89.000 |
| Potato starch | 7.19 | 7.19 |
| Gelatine | 1.000 | 1.000 |
| Stearic acid | 1.000 | 1.000 |
| Talc | 1.000 | 1.000 |
| Purified water** | q.s | - |
| ***Total tablet weight [mg]:*** | **100.00** | **100.00** |

The concentrations may be altered to change certain properties of the formulations, for instance, the dissolution profile. Table 4 shows the ranges for each ingredient.

| **Ingredient** | **Range** |
|---|---|
| Colchicine | 0.25-0.75 % |
| Salicylic acid | 0.1-0.3 % |
| Lactose monohydrate | 10-95 % |
| Potato starch | 5-50 % |
| Gelatine | 0.5-5.0 % |
| Stearic acid | 0.5-5.0 % |
| Talc | 0.5-5.0 % |
| Purified water** | q.s |
| ***Total tablet weight*** | **50-200 mg** |

### Example 3: Colchicine-salicylate Immediate Release Tablet

Alternatively, salicylic acid may be added in excess, resulting in colchicine-salicylate and free salicylic acid. An example of a colchicine-salicylate immediate Release Tablet with a final content of 0.500mg colchicine-salicylate is shown in Table 5 below.

| **Ingredient** | **Ingredients in mg per 100mg** | **Composition in mg/Tablet** |
|---|---|---|
| Colchicine | 0.372 | - |
| Salicylic acid | 0.500 | 0.372 |
| Colchicine-salicylate* | | 0.500 |
| Lactose monohydrate | 89.000 | 89.000 |
| Potato starch | 7.128 | 7.128 |
| Gelatine | 1.000 | 1.000 |
| Stearic acid | 1.000 | 1.000 |
| Talc | 1.000 | 1.000 |
| Purified water** | q.s | - |
| ***Total tablet weight [mg]:*** | **100.00** | **100.00** |

| | | |
|---|---|---|
| *equals 0.372mg colchicine ** used as granulating fluid, present in finished product with less than 4% | | |

The concentrations may be altered to change certain properties of the formulations, for instance, the dissolution profile. Table 6 shows the ranges for each ingredient.

| **Ingredient** | **Range** | **Function** |
|---|---|---|
| Colchicine | 0.25-0.75 % | Active Pharmaceutical Ingredient (API) |
| Salicylic acid | 0.3-1.0 % | |
| Lactose monohydrate | 10-95 % | Filling Agent |
| Potato starch | 5-50 % | Disintegrant |
| Gelatine | 0.5-5.0 % | Granulation Agent |
| Stearic acid | 0.5-5.0 % | Lubricant |
| Talc | 0.5-5.0 % | Glidant |
| Purified water** | q.s | Diluent for API and Binder |
| ***Total tablet weight*** | **50-200 mg** | |

| | | |
|---|---|---|
| **used as granulating fluid, present in finished product with less than 4% | | |

### Example 4: Colchicine-salicylate Sustained Release Tablet

This example illustrates a colchicine-salicylate sustained release tablet. The tablet uses the ingredients and concentrations shown in Table 7 below.

| **Ingredient** | **mg / Tablet** | **Tablet %** | **Function** |
|---|---|---|---|
| Colchicine-salicylate | 0.675 | 0.675 | Active Pharmaceutical Ingredient (API) |
| Lactose monohydrate | 58.825 | 58.8 | Filling Agent |
| Pregelatinized Starch | 7.50 | 7.5 | Filling Agent |
| Hypromellose 6mPa*s | 1.000 | 1.0 | Binder |
| Purified water** | q.s. | q.s. | Diluent for API and Binder |
| Lactose monohydrate | 5.00 | 5.00 | Filling Agent |
| Retalac ( Compound of Lactose monohydrate and Hypromellose 4000mPa*s 50/50 w/w %) | 25.00 | 25.00 | Retarding Agent |
| Talc | 1.00 | 1.0 | Glidant |
| Stearic acid 50 | 1.00 | 1.0 | Lubricant |
| ***Total tablet weight [mg]:*** | **100.00** | | |

| | | | |
|---|---|---|---|
| ** used as granulating fluid, present in finished product with less than 4% | | | |

The concentrations may be altered to change certain properties of the formulations, for instance, the dissolution profile. Table 8 shows the ranges for each ingredient.

| **Ingredient** | **Range** | **Function** |
|---|---|---|
| Colchicine-salicylate | 0.5-1.0 | Active Pharmaceutical Ingredient (API) |
| Lactose monohydrate | 10-80 | Filling Agent |
| Pregelatinized Starch | 5-50 | Filling Agent |
| Hypromellose 6mPa*s | 0.1-2.0 | Binder |
| Purified water** | q.s. | Diluent for API and Binder |
| Lactose monohydrate | 10-30 | Filling Agent |
| Retalac ( Compound of Lactose monohydrate and Hypromellose 4000mPa*s 50/50 w/w %) | 10-40 | Retarding Agent |
| Talc | 0.5-5 | Glidant |
| Stearic acid 50 | 0.5-5 | Lubricant |
| ***Total tablet weight [mg]:*** | | |

| | | |
|---|---|---|
| ** used as granulating fluid, present in finished product with less than 4% | | |

Any possible changes to the manufacturing process as exemplified in examples 2 and 3 are also possible for the manufacturing of a Colchicine-salicylate sustained release tablet, making any necessary adjustments in the amount of colchicine, salicylic acid, lactose monohydrate or Pregelatinized Starch in table 8 in accordance with tables 3 and 5.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims and list of embodiments disclosed herein. Although specific terms are employed herein, they are used in a generic and descriptive sense only and not for purposes of limitation.
1. A method for treating and/or preventing cardiovascular disease in a subject by administering to the subject a therapeutically effective amount of a composition comprising no more than about 1.0 or about 0.81 total mg of colchicine salicylate, thereby treating and/or preventing the cardiovascular disease in the subject.
2. The method of claim 1, wherein the composition is administered once a day.
3. The method of claim 1 or 2, wherein the composition is administered orally, topically, parenterally, intraorbitally, ophthalmically, intraventricularly, intracranially, intracapsularly, intraspinally, intracisternally, intraperitoneally, buccally, rectally, vaginally, intranasally, or by aerosol administration and/or inhalation spray or via an implanted reservoir.
4. The method of any one of claims 1 to 3, wherein the composition is administered in the form of a tablet, capsule, liquid dose, gel or powder.
5. The method of any one of claims 1 to 4, wherein the composition is an immediate release composition or a sustained release composition.
6. The method of any one of claims 1 to 5, wherein the cardiovascular disease is selected from the group consisting of acute pericarditis, recurrent pericarditis, post-pericardiotomy syndrome (PPS) and cardiovascular events in patients with stable coronary disease.
7. The method of any one of claims 1 to 5, wherein the cardiovascular disease is a coronary disease.
8. The method of any one of claims 1 to 5, for use in combination with conventional therapy for the long-term prevention of an acute cardiovascular event in patients with established stable coronary disease.
9. The method of any one of claims 1 to 5, wherein the cardiovascular event is acute coronary syndrome, out-of-hospital cardiac arrest or noncardioembolic ischemic stroke.
10. The method of any one of claims 1 to 9, further comprising coadministering to the subject a second agent for the treatment and/or prevention of the cardiovascular event in the subject.
11. The method of any one of claims 1 to 10, wherein the second agent is a colchicine-compatible statin.
12. The method of any one of claims 1 to 11, wherein the colchicine-compatible statin is selected from the group consisting of atorvastatin, fluvastatin, lovastatin, pitavastatin, rosuvastatin, simvastatin and pravastatin, a derivative or a salt thereof.
13. The method of claim 10, wherein the second agent is an anti-platelet agent.
14. The method of claim 13, wherein the anti-platelet agent is selected from the group consisting of irreversible cyclooxygenase inhibitors; adenosine diphosphate (ADP) receptor inhibitors; phosphodiesterase inhibitors; glycoprotein IIB/IIIA inhibitors; adenosine reuptake inhibitors; thromboxane inhibitors; and Thromboxane receptor antagonists.
15. The method of any one of claims 1 to 14, wherein the composition comprises a pharmaceutically acceptable carrier, diluent or excipient.
16. A method for treating and/or preventing inflammatory disease by administering to the subject a therapeutically effective amount of a composition comprising no more than about 0.81 total mg of colchicine salicylate, thereby treating and/or preventing inflammatory disease in the subject.
17. The method of claim 16, wherein the inflammatory disease is selected from the group consisting of gout, familial Mediterranean fever, Behcet's disease, Age-related macular degeneration and Alzheimer's disease.
18. The method of any one of claims 16 and 17, wherein the composition is administered once a day or at least once a day.
19. The method of any one of claims 16 to 18, wherein the composition is administered orally, topically, parenterally, intraorbitally, ophthalmically, intraventricularly, intracranially, intracapsularly, intraspinally, intracisternally, intraperitoneally, buccally, rectally, vaginally, intranasally, or by aerosol administration and/or inhalation spray or via an implanted reservoir.
20. The method of any one of claims 16 to 19, wherein the composition is administered in the form of a tablet, capsule, liquid dose, gel or powder.
21. The method of any one of claims 16 to 20, wherein the composition is an immediate release composition or a sustained release composition.
22. The method of any one of claims 16 to 21, wherein the composition comprises a pharmaceutically acceptable carrier, diluent or excipient.
23. A therapeutically effective composition comprising no more than about 0.675 total mg of colchicine salicylate and at least one of a pharmaceutically acceptable carrier, diluent or excipient.
24. The composition of claim 23, wherein the composition is formulated for administration once a day or at least once a day.
25. The composition of claim 23 or 24, wherein the composition is formulated for administration orally, topically, parenterally, intraorbitally, ophthalmically, intraventricularly, intracranially, intracapsularly, intraspinally, intracisternally, intraperitoneally, buccally, rectally, vaginally, intranasally, or by aerosol administration and/or inhalation spray or via an implanted reservoir.
26. The composition of any one of claims 23 to 25, wherein the composition is administered in the form of a tablet, capsule, liquid dose, gel or powder.
27. The composition of any one of claims 23 to 26, wherein the composition is an immediate release composition.
28. The composition of any one of claims 23 to 27, wherein the composition releases at least about 80% of the colchicine salicylate in vitro within 0.5 hours, 1.0 hours, 1.5, hours, 2.0 hours, 2.5 hours, or within 3.0 hours.
29. The composition of any one of claims 23 to 28, wherein the pharmaceutically acceptable excipient is selected from a group consisting of a disintegrant, a granulation agent, a filling agent, a glidant, and a lubricant, or a combination thereof.
30. The composition of any one of claims 23 to 29, wherein the pharmaceutically acceptable excipient is a disintegrant selected from the group consisting of sucrose, lactose, lactose monohydrate, trehalose, maltose, mannitol, sorbitol, croscarmellose sodium, crospovidone, alginic acid, sodium alginate, methacrylic acid DVB, cross-linked PVP, microcrystalline cellulose, polacrilin potassium, sodium starch glycolate, starch or starch derivatives such as sodium starch glycolate, pregelatinised starch, maize starch, potato starch, rice starch, wheat starch, mixturmicrocrystalline cellulose, cross-linked polyvinylpyrrolidone, cross-linked sodium carboxymethylcellulose, and mixtures thereof.
31. The composition of any one of claims 23 to 30, wherein the total amount of distintegrant agent in the composition is between about 5.0 wt % and 50.0 wt % of the composition.
32. The composition of any one of claims 23 to 31, wherein the pharmaceutically acceptable excipient is a granulation agent selected from the group consisting of gelatin, povidone, starch, starch paste, cellulose derivatives, HPMC, hydroxypropyl cellulose, acacia, sodium alginate, guar, siliceous material, water-soluble polymeric materials, polyethylene glycols, PEG400, polyvinylpyrrolidone, ethanol, isopropanol and Pluronic L44 and combinations thereof.
33. The composition of any one of claims 23 to 32, wherein the total amount of granulation agent in the formulation is between about 0.5 wt % and 5.0 wt % of the formulation.
34. The composition of any one of claims 23 to 33, wherein the pharmaceutically acceptable excipient is a filling agent selected from the group consisting of sucrose, lactose, lactose monohydrate, trehalose, maltose, mannitol and sorbitol, croscarmellose sodium, crospovidone, alginic acid, sodium alginate, methacrylic acid DVB, cross-linked PVP, microcrystalline cellulose, polacrilin potassium, sodium starch glycolate, starch, pregelatinized starch, and combinations thereof.
35. The composition of any one of claims 23 to 34, wherein the total amount of filling agent in the formulation is between about 10.0 wt % and 95.0 wt % of the formulation.
36. The composition of any one of claims 23 to 35, wherein the pharmaceutically acceptable excipient is a glidant selected from the group consisting of colloidal silicon dioxide, magnesium trisilicate, powdered cellulose, talc, and tribasic calcium phosphate
37. The composition of any one of claims 23 to 36, wherein the total amount of glidant in the formulation is between about 0.5 wt % to about 5 wt % of the formulation.
38. The composition of any one of claims 23 to 37 wherein the pharmaceutically acceptable excipient is a lubricant selected from the group consisting of glyceryl behenate, stearic acid, hydrogenated vegetable oils, stearyl alcohol, leucine, polyethylene glycol, magnesium stearate, glyceryl monostearate, polyethylene glycol, ethylene oxide polymers, sodium lauryl sulfate, magnesium lauryl sulfate, sodium oleate, sodium stearyl fumarate, DL-leucine, and colloidal silica.
39. The composition of any one of claims 23 to 38, wherein the lubricant is included in an amount between about 0.5 wt % to about 5 wt % of the formulation.
40. The composition of any one of claims 23 to 26, wherein the composition is a sustained release composition.
41. The composition of any one of claims 23 to 26 and 40, wherein the composition releases at least about 80% of the colchicine in vitro within 24 hours, within 16 hours, within 12 hours, within 8 hours, within 6 hours, within 4 hours, within 3.5 hours, within 1.5 hours, or within 1 hour.
42. The composition of any one of claims 23 to 26 and 40 to 41, wherein the composition releases at least about 20% of the colchicine in vitro within the first 30 minutes.
43. The composition of any one of claims 23 to 26 and 40 to 42, wherein the composition further comprising a retarding agent.
44. The composition of any one of claims 23 to 26 and 40 to 43, wherein the retarding agent is selected from a group consisting of cellulose ethers, cellulose esters, acrylic acid copolymers, waxes, gums, glyceryl fatty acid esters and sucrose fatty acid esters.
45. The composition of any one of claims 23 to 26 and 40 to 44, wherein the retarding agent contains hypromellose.
46. The composition of any one of claims 23 to 26 and 40 to 45, wherein the retarding agent is included in an amount between about 5 wt % and about 40 wt % of the formulation
47. The composition of any one of claims 23 to 26 and 40 to 46, wherein the pharmaceutically acceptable excipient is selected from a group consisting of a binder, a filling agent, a glidant, and a lubricant, or a combination thereof.
48. The composition of any one of claims 23 to 26 and 40 to 47, wherein the pharmaceutically acceptable excipient is a binder selected from the group consisting of starches, gelatin, polyvinylpyrrolidone, cellulose derivatives, polyvinyl alcohol and mixtures thereof.
49. The composition of any one of claims 23 to 26 and 40 to 48, wherein the cellulose derivative is selected from a group consisting of hydroxypropyl methylcellulose (HPMC) and hydroxypropyl cellulose (HPC).
50. The composition of any one of claims 23 to 26 and 40 to 49, wherein the cellulose derivative forms a hydrophilic matrix.
51. The composition of any one of claims 23 to 26 and 40 to 50, wherein the pharmaceutically acceptable excipients is a filling agent selected from the group consisting of sucrose, lactose, in particular lactose monohydrate, trehalose, maltose, mannitol and sorbitol, croscarmellose sodium, crospovidone, alginic acid, sodium alginate, methacrylic acid DVB, cross-linked PVP, microcrystalline cellulose, polacrilin potassium, sodium starch glycolate, starch, pregelatinized starch, and combinations thereof.
52. The composition of any one of claims 23 to 26 and 40 to 51, wherein the total amount of filling agent in the formulation is between about 5.0 wt % and 80.0 wt % of the composition.
53. The composition of any one of claims 23 to 26 and 40 to 52, wherein the pharmaceutically acceptable excipient is a glidant selected from the group consisting of colloidal silicon dioxide, magnesium trisilicate, powdered cellulose, talc, and tribasic calcium phosphate.
54. The composition of any one of claims 23 to 26 and 40 to 53, wherein total amount of glidant in the formulation is between about 0.5 wt % to about 5 wt % of the formulation.
55. The composition of any one of claims 23 to 26 and 40 to 54, wherein the pharmaceutically acceptable excipient is a lubricant selected from the group consisting of glyceryl behenate, stearic acid, hydrogenated vegetable oils, stearyl alcohol, leucine, polyethylene glycol, magnesium stearate, glyceryl monostearate, polyethylene glycol, ethylene oxide polymers, sodium lauryl sulfate, magnesium lauryl sulfate, sodium oleate, sodium stearyl fumarate, DL-leucine, and colloidal silica.
56. The composition of any one of claims 23 to 26 and 40 to 55, wherein the lubricant is included in an amount between about 0.5 wt % to about 5 wt % of the formulation.
57. The composition of any one of claims 23 to 56, wherein the composition is in a dosage form selected from the group consisting of a tablet, a pill, a capsule, a caplet, a suppository, a dermal patch, a cream, sublingual formulation, eye drops, gel, ointment, a troche, a pouch, sprinkles, or in fixed combination with a surgically insertable medical device.
58. The composition of any one of claims 23 to 57 for use in preventing and/or treating a cardiovascular disease.
59. The composition of claim 58, wherein the cardiovascular disease is selected from the group consisting of acute pericarditis, recurrent pericarditis, post-pericardiotomy syndrome (PPS) and cardiovascular events in patients with stable coronary disease.
60. The composition of any one of claims 23 to 58, for use in combination with conventional therapy for the long-term prevention of an acute cardiovascular event in patients with established stable coronary disease.
61. The composition of claim 60, wherein said acute cardiovascular event is acute coronary syndrome, out-of-hospital cardiac arrest or noncardioembolic ischemic stroke.
62. The composition of claim 60 or 61, wherein the conventional therapy includes the administration of a colchicine-compatible statin.
63. The composition of claim 62, wherein the colchicine-compatible statin is administered in a fixed or unfixed combination with colchicine salicylate.
64. The composition of claim 62 or 63, wherein the colchicine-compatible statin is selected from the group consisting of atorvastatin, rosuvastatin, simvastatin and pravastatin, a derivative or a salt thereof.
65. The composition of any one of claims 23 to 57 for use in preventing and/or treating an inflammatory disease.
66. The composition of claim 65, wherein the inflammatory disease is selected from the group consisting of gout, familial Mediterranean fever, Behcet's disease, Age-related macular degeneration and Alzheimer's disease.
67. The composition of any one of claims 23 to 66, wherein the total daily dose of colchicine administered to a subject is no more than about 0.5 mg, 0.60 mg, 0.675 mg or 1.0 mg.
68. A process of preparing a colchicine salicylate composition comprising:
   (A) forming a granulate by dissolving about 0.25 to about 0.75 % weight of the total composition of colchicine salicylate in an acceptable solvent and
   (B) adding a binder and a first filling agent to the granulate of Step A, and forming a wet granulate;
   (C) drying the wet granulate of Step B;
   (D) blending the dried granulate from Step C with a retarding agent, a second filling agent, a glidant and a lubricant; and
   (E) compressing the final granulation from step D into a tablet.
69. The process of claim 68 wherein:
   in Step B the binder is Hypromellose;
   in Step B the first filling agent used is lactose monohydrate and Pregelatinized Starch; and
   in Step D the retarding agent used is Retalac; the second filling agent used is lactose monohydrate, the glidant used is Talc, and the lubricant used is Stearic acid.
70. A tablet prepared by the method of claim 68 or 69.
71. A tablet comprising,
   i. colchicine salicylate from about 0.5 wt% to about 1.0 wt%;
   ii. a filling agent from about 10 wt% to about 95 wt%;
   iii. a disintegrant from about 5 wt% to about 50 wt%;
   iv. a granulation agent from about 0.5 wt% to about 5.0 wt%;
   v. a lubricant from about 0.5 wt% to about 5.0 wt%; and
   vi. a glidant from about 0.5 wt% to about 5.0 wt%;
   wherein said tablet exhibits an increase in bioavailability of said colchicine salicylate.
72. The tablet of claim 71, comprising
   i. colchicine salicylate from about 0.6 wt% to about 0.9 wt%;
   ii. a filling agent from about 85 wt% to about 95 wt%;
   iii. a disintegrant from about 5 wt% to about 10 wt%;
   iv. a granulation agent from about 0.5 wt% to about 5.0 wt%;
   v. a lubricant from about 0.5 wt% to about 1.5 wt%; and
   vi. a glidant from about 0.5 wt% to about 1.5 wt%.
73. The tablet of claim 74, comprising
   i. colchicine salicylate at about 0.675 wt% or at about 0.81 wt%;
   ii. a filling agent at about 89 wt%;
   iii. a disintegrant from about 6.9 wt% to about 7.5 wt%;
   iv. a granulation agent at about 1.0 wt%;
   v. a lubricant at about 1.0 wt%%; and
   vi. a glidant at about 1.0 wt%.
74. The tablet of claim 71, 72 or 73, wherein said increase in bioavailability is an increase in Cmax.
75. The tablet of claim 71, 72 or 73, wherein said increase in bioavailability is an increase in AUC.
76. The tablet of claim 71, 72 or 73, wherein said filling agent is selected from the group consisting of sucrose, lactose, in particular lactose monohydrate, trehalose, maltose, mannitol and sorbitol, croscarmellose sodium, crospovidone, alginic acid, sodium alginate, methacrylic acid DVB, cross-linked PVP, microcrystalline cellulose, polacrilin potassium, sodium starch glycolate, starch, pregelatinized starch, and combinations thereof.
77. The tablet of claim 76, wherein said filling agent is lactose monohydrate.
78. The tablet of claim 71, 72 or 73, wherein said disintegrant is selected from the group consisting of sucrose, lactose, lactose monohydrate, trehalose, maltose, mannitol, sorbitol, croscarmellose sodium, crospovidone, alginic acid, sodium alginate, methacrylic acid DVB, cross-linked PVP, microcrystalline cellulose, polacrilin potassium, sodium starch glycolate, starch or starch derivatives such as sodium starch glycolate, pregelatinised starch, maize starch, potato starch, rice starch, wheat starch, mixturmicrocrystalline cellulose, cross-linked polyvinylpyrrolidone, cross-linked sodium carboxymethylcellulose, or mixtures thereof.
79. The tablet of claim 78, wherein said disintegrants is potato starch.
80. The tablet of claim 71, 72 or 73, wherein said granulation agent is selected from the group consisting of gelatin, povidone, starch, starch paste, cellulose derivatives, HPMC, hydroxypropyl cellulose, acacia, sodium alginate, guar, siliceous material, water-soluble polymeric materials, polyethylene glycols, PEG400, polyvinylpyrrolidone, ethanol, isopropanol and Pluronic L44.
81. The tablet of claim 80, wherein said granulation agent is gelatin.
82. The tablet of claim 71, 72 or 73, wherein said lubricant is selected from the group consisting of glyceryl behenate, stearic acid, hydrogenated vegetable oils, stearyl alcohol, leucine, polyethylene glycol, magnesium stearate, glyceryl monostearate, polyethylene glycol, ethylene oxide polymers, sodium lauryl sulfate, magnesium lauryl sulfate, sodium oleate, sodium stearyl fumarate, DL-leucine, and colloidal silica.
83. The tablet of claim 82, wherein said lubricant is stearic acid.
84. The tablet of claim 71, 72 or 73, wherein said glidant is selected from the group consisting of colloidal silicon dioxide, magnesium trisilicate, powdered cellulose, talc, and tribasic calcium phosphate.
85. The tablet of claim of claim 84, wherein said glidant is talc.
86. The tablet of claim 71, 72 or 73, comprising
   i. colchicine salicylate at about 0.675 wt% or at about 0.81 wt%;
   ii. lactose monohydrate at about 89 wt%;
   iii. potato starch from about 6.9 wt% to about 7.5 wt%;
   iv. gelatin at about 1.0 wt%;
   v. stearic acid at about 1.0 wt%%; and
   vi. talc at about 1.0 wt%.
87. A method of treating a cardiovascular or inflammatory disease comprising, administering an effective amount of colchicine salicylate in the form of a tablet of claim 71, 72, 73 or 86 to a subject in need thereof.
88. The composition of claim 23, wherein said composition exhibits an increase in bioavailability of said colchicine salicylate.
89. A tablet comprising,
   i. colchicine salicylate from about 0.5 wt% to about 1.0 wt%;
   ii. a filling agent(s) from about 10 wt% to about 95 wt%;
   iii. a binder from about 0.1 to about 2.0%;
   iv. a retarding agent from about 5.0 wt% to about 40.0 wt%;
   v. a lubricant from about 0.5 wt% to about 5.0 wt%; and
   vi. a glidant from about 0.5 wt% to about 5.0 wt%;
   wherein said tablet exhibits an increase in bioavailability of said colchicine salicylate and wherein said tablet exhibits sustained release of said colchicine salicylate.
90. The tablet of claim 89, comprising
   i. colchicine salicylate at about 0.675 wt%;
   ii. a filling agent(s) at about 70 wt%;
   iii. a binder at about 1.0 wt%;
   iv. a retarding agent at about 25.0 wt%;
   v. a lubricant at about 1.0 wt%; and
   vi. a glidant at about 1.0 wt%.
91. The tablet of claim 90, comprising
   i. colchicine salicylate at about 0.675 wt%;
   ii. lactose monohydrate at about 63 wt%;
   iii. pregelatinized starch at about 7.5 wt%;
   iv. hypromellose at about 1.0 wt%;
   iv. Retalac at about 25.0 wt%;
   v. stearic acid 50 at about 1.0 wt%; and
   vi. talc at about 1.0 wt%.
92. The tablet of claim 89, 90 or 91, wherein said increase in bioavailability is an increase in Cmax.
93. The tablet of claim 89, 90 or 91, wherein said increase in bioavailability is an increase in AUC.
94. A method of treating a cardiovascular or inflammatory disease comprising, administering an effective amount of colchicine salicylate in the form of a tablet of claim 89, 90 or 91 to a subject in need thereof.

## Claims

1. A therapeutically effective amount of a composition comprising a daily dosage amount of about 1.0 mg or about 0.81 total mg of colchicine salicylate for use in treating and/or preventing a cardiovascular disease in a subject, or for use in treating and/or preventing inflammatory disease in a subject, wherein:
(a) the cardiovascular disease is acute pericarditis, recurrent pericarditis, post-pericardiotomy syndrome (PPS), or a cardiovascular event in a subject with stable coronary disease, and wherein the cardiovascular event is acute coronary syndrome, out-of-hospital cardiac arrest or noncardioembolic ischemic stroke; and
(b) the inflammatory disease is gout, familial Mediterranean fever, Behcet's disease, Age-related macular degeneration, or Alzheimer's disease.

2. A therapeutically effective composition comprising a daily dose amount of no more than about 0.675 total mg of colchicine salicylate and at least one of a pharmaceutically acceptable carrier, diluent, or excipient.

3. A therapeutically effective amount of a composition comprising a daily dosage amount of about 1.0 mg or about 0.81 total mg of colchicine salicylate for use according to claim 1, wherein, in use, the composition is administered 1 to 10 times per day.

4. A therapeutically effective amount of a composition comprising a daily dosage amount of about 1.0 mg or about 0.81 total mg of colchicine salicylate for use according to claim 1, wherein, in use, the composition is administered orally, topically, parenterally, intraorbitally, ophthalmically, intraventricularly, intracranially, intracapsularly, intraspinally, intracisternally, intraperitoneally, buccally, rectally, vaginally, intranasally, or by aerosol administration and/or inhalation spray or via an implanted reservoir.

5. A therapeutically effective amount of a composition comprising a daily dosage amount of about 1.0 mg or about 0.81 total mg of colchicine salicylate for use according to claim 1; or a therapeutically effective composition according to claim 2, in use, wherein the composition is administered in the form of a tablet, capsule, liquid dose, gel or powder.

6. A therapeutically effective amount of a composition comprising a daily dosage amount of about 1.0 mg or about 0.81 total mg of colchicine salicylate for use according to claim 1; or a therapeutically effective composition according to claim 2, wherein the composition is an immediate release composition or a sustained release composition.

7. A therapeutically effective amount of a composition comprising a daily dosage amount of about 1.0 mg or about 0.81 total mg of colchicine salicylate for use according to claim 1; or a therapeutically effective composition according to claim 2, wherein the composition further comprises a second agent for the treatment and/or prevention of the cardiovascular event in the subject and wherein the second agent is:
(a) a colchicine-compatible statin selected from atorvastatin, fluvastatin, lovastatin, pitavastatin, rosuvastatin, simvastatin, pravastatin, and combinations thereof; and/or
(b) an anti-platelet agent selected from an irreversible cyclooxygenase inhibitor, an adenosine diphosphate (ADP) receptor inhibitor, a phosphodiesterase inhibitor, a glycoprotein IIB/IIIA inhibitor, an adenosine reuptake inhibitor, a thromboxane inhibitor, a thromboxane receptor antagonist, and combinations thereof.

8. A therapeutically effective composition according to claim 2, wherein:
(a) the composition releases at least about 80% of the colchicine salicylate *in vitro* within 0.5 hours, 1.0 hours, 1.5, hours, 2.0 hours, 2.5 hours, or within 3.0 hours;
(b) the composition releases at least about 80% of the colchicine *in vitro* within 24 hours, within 16 hours, within 12 hours, within 8 hours, within 6 hours, within 4 hours, within 3.5 hours, within 1.5 hours, or within 1 hour; and/or
(c) the composition releases at least about 20% of the colchicine *in vitro* within the first 30 minutes.

9. A therapeutically effective amount of a composition comprising a daily dosage amount of about 1.0 mg or about 0.81 total mg of colchicine salicylate for use according to claim 1, wherein the composition further comprises a pharmaceutically acceptable carrier, diluent, and/or excipient.

10. A therapeutically effective amount of a composition comprising a daily dosage amount of about 1.0 mg or about 0.81 total mg of colchicine salicylate for use according to any one of claims 1 and 3 to 9; or a therapeutically effective composition according to any one of claims 2 to 8, wherein the pharmaceutically acceptable excipient is one or more of a disintegrant, a granulation agent, a filling agent, a glidant, a binder, and a lubricant, and wherein:
(a) the disintegrant is one or more of sucrose, lactose, lactose monohydrate, trehalose, maltose, mannitol, sorbitol, croscarmellose sodium, crospovidone, alginic acid, sodium alginate, methacrylic acid divinylbenzene (DVB), cross-linked polyvinylpyrrolidone (PVP), microcrystalline cellulose, polacrilin potassium, sodium starch glycolate, starch or starch derivatives such as sodium starch glycolate, pregelatinised starch, maize starch, potato starch, rice starch, wheat starch, mixturmicrocrystalline cellulose, cross-linked polyvinylpyrrolidone, and cross-linked sodium carboxymethylcellulose;
(b) the granulation agent is one or more of gelatin, povidone, starch, starch paste, cellulose derivatives, hydroxypropyl methyl cellulose (HPMC), hydroxypropyl cellulose, acacia, sodium alginate, guar, siliceous material, water-soluble polymeric materials, polyethylene glycols, polyethylene glycol (PEG) 400, polyvinylpyrrolidone, ethanol, isopropanol, and Pluronic L44;
(c) the filling agent is one or more of sucrose, lactose, lactose monohydrate, trehalose, maltose, mannitol, sorbitol, croscarmellose sodium, crospovidone, alginic acid, sodium alginate, methacrylic acid DVB, cross-linked PVP, microcrystalline cellulose, polacrilin potassium, sodium starch glycolate, starch, and pregelatinized starch;
(d) the glidant is one or more of colloidal silicon dioxide, magnesium trisilicate, powdered cellulose, talc, and tribasic calcium phosphate;
(e) the binder is one or more of starches, gelatin, polyvinylpyrrolidone, cellulose derivatives, and polyvinyl alcohol; and
(f) the lubricant is one or more of glyceryl behenate, stearic acid, hydrogenated vegetable oils, stearyl alcohol, leucine, polyethylene glycol, magnesium stearate, glyceryl monostearate, polyethylene glycol, ethylene oxide polymers, sodium lauryl sulfate, magnesium lauryl sulfate, sodium oleate, sodium stearyl fumarate, DL-leucine, and colloidal silica.

11. A therapeutically effective amount of a composition comprising a daily dosage amount of about 1.0 mg or about 0.81 total mg of colchicine salicylate for use according to claim 9 or 10; or a therapeutically effective composition according to claim 10, wherein the binder is a cellulose derivate selected from hydroxypropyl methylcellulose (HPMC), hydroxypropyl cellulose (HPC), and a combination thereof, and wherein the binder forms a hydrophilic matrix.

12. A therapeutically effective amount of a composition comprising a daily dosage amount of about 1.0 mg or about 0.81 total mg of colchicine salicylate for use according to any one of claims 9 to 11; or a therapeutically effective composition according to claim 10 or 11, wherein the composition is a tablet and exhibits an increase in bioavailability of the colchicine salicylate, and wherein:
(a) the total amount of colchicine salicylate in the composition is between about 0.5 wt% to about 1.0 wt% of the composition, the total amount of filling agent in the composition is between about 10 wt% to about 95 wt% of the composition, the total amount of disintegrant in the composition is between about 5 wt% to about 50 wt% of the composition, the total amount of granulation agent in the composition is between about 0.5 wt% to about 5.0 wt% of the composition, the total amount of lubricant in the composition is between about 0.5 wt% to about 5.0 wt% of the composition, and the total amount of glidant in the composition is between about 0.5 wt% to about 5.0 wt% of the composition; or
(b) the total amount of colchicine salicylate in the composition is between about 0.6 wt% to about 0.9 wt% of the composition, the total amount of filling agent in the composition is between about 85 wt% to about 95 wt% of the composition, the total amount of disintegrant in the composition is between about 5 wt% to about 10 wt% of the composition, the total amount of granulation agent in the composition is between about 0.5 wt% to about 5.0 wt% of the composition, the total amount of lubricant in the composition is between about 0.5 wt% to about 1.5 wt% of the composition, and the total amount of glidant in the composition is between about 0.5 wt% to about 1.5 wt% of the composition.

13. A therapeutically effective amount of a composition comprising a daily dosage amount of about 1.0 mg or about 0.81 total mg of colchicine salicylate for use according to any one of claims 9 to 12; or a therapeutically effective composition according to any one of claims 10 to 12, wherein the composition is a tablet and increased bioavailability in colchicine salicylate is an increase in Cmax or and/or an increase in AUC, and wherein:
(a) colchicine salicylate is present at about 0.675 wt% or at about 0.81 wt% of the composition, the filling agent is lactose monohydrate and is present at about 89 wt% of the composition, the disintigrant is potato starch and is present from about 6.9 wt% to about 7.5 wt% of the composition, the granulating agent is gelatin and is present at about 1.0 wt% of the composition, the lubricant is stearic acid and is present at about 1.0 wt% of the composition, and the glidant is talc and is present at about 1.0 wt% of the composition;
(b) colchicine salicylate is present from about 0.6 wt% to about 0.9 wt% of the composition, the filling agent is present from about 85 wt% to about 95 wt% of the composition, the disintegrant is present from about 5 wt% to about 10 wt% of the composition, the granulation agent is present from about 0.5 wt% to about 5.0 wt% of the composition, the lubricant is present from about 0.5 wt% to about 1.5 wt% of the composition, and the glidant is present from about 0.5 wt% to about 1.5 wt% of the composition; or
(c) colchicine salicylate is present from about 0.675 wt% to about 0.81 wt% of the composition, the filling agent is present at about 89 wt% of the composition, the disintegrant is present from about 6.9 wt% to about 7.5 wt% of the composition, the granulation agent is present at about 1.0 wt% of the composition, the lubricant is present at about 1.0 wt% of the composition, and the glidant is present at about 1.0 wt% of the composition.

14. A therapeutically effective amount of a composition comprising a daily dosage amount of about 0.1 wt% to about 1.0 wt% of colchicine salicylate for use according to any one of claims 1 and 3 to 13; or a therapeutically effective composition according to any one of claims 2 to 8 and 9 to 13, wherein the composition is a sustained release composition further comprising a retarding agent, wherein the retarding agent is one or more of cellulose ethers, cellulose esters, acrylic acid copolymers, waxes, gums, glyceryl fatty acid esters, and sucrose fatty acid esters, and wherein the total amount of the retarding agent in the composition is between about 5 wt% and about 40 wt% of the composition.

15. A therapeutically effective amount of a composition comprising a daily dosage amount of about 1.0 mg or about 0.81 total mg of colchicine salicylate for use according to claim 14; or a therapeutically effective composition according to claim 14, wherein the retarding agent is approximately equal portions of HPMC having a viscosity of 4000 mPa•s and lactose monohydrate, and wherein the retarding agent is present in the composition from about 5.0 wt% to about 40.0 wt%, or at about 25 wt%, of the composition.

16. A process of preparing a colchicine salicylate composition, which comprises:
(a) forming a granulate by dissolving about 0.25 to about 0.75 % weight of the total composition of colchicine salicylate in an acceptable solvent;
(b) adding a binder and a first filling agent to the granulate of Step A, and forming a wet granulate;
(c) drying the wet granulate of Step B;
(d) blending the dried granulate from Step C with a retarding agent, a second filling agent, a glidant and a lubricant; and
(e) compressing the final granulation from step D into a tablet wherein:
(i) in Step B the binder is hydroxypropyl methyl cellulose (HPMC);
(ii) in Step B the first filling agent is lactose monohydrate and pregelatinized starch; and
(iii) in Step D the retarding agent is approximately equal portions of HPMC having a viscosity of 4000 mPa•s and lactose monohydrate, the second filling agent is lactose monohydrate, the glidant is talc, and the lubricant is stearic acid.
